# EUROPEAN PATENT APPLICATION

(11) **EP 4 345 102 A1**
(43) Date of publication of application: **03.04.2024**
(21) Application number: 22199174.8
(22) Date of filing: 30.09.2022
(51) Int. Cl.: C07D 487/04, C07C 243/00, C07D 231/38

(54) **IMPROVED SYNTHESIS OF OTVICICLIB**

(71) Applicant: Medizinische Universität Innsbruck, 6020 Innsbruck (AT)
(72) Inventor: HUBER, Lukas A., 6130 Schwaz (AT); GSTACH, Hubert, 1070 Wien (AT); MASTALIR, Matthias, 1120 Wien (AT); LEBAN, Johann, 6080 Innsbruck (AT)
(74) Representative: Vossius & Partner Patentanwälte Rechtsanwälte mbB

(57) **Abstract**

The present invention relates to an improved synthesis of Otviciclib (N-{4-[1-(2,6-Dichloro-4-sulfamoyl-phenyl)-3-dimethylamino-4-oxo-4,5-dihydro-1H-pyrazolo[3,4-d]pyrimidin-6-ylmethyl]-phenyl}-2-diethylamino-acetamide) and its derivatives, and furthermore relates to intermediates useful in this synthesis.

## Description

The present invention relates to an improved synthesis of Otviciclib (N-{4-[1-(2,6-Dichloro-4-sulfamoyl-phenyl)-3-dimethylamino-4-oxo-4,5-dihydro-1H-pyrazolo[3,4-d]pyrimidin-6-ylmethyl]-phenyl}-2-diethylamino-acetamide) and its derivatives, and furthermore relates to intermediates useful in this synthesis.

Otviciclib and its derivatives are particularly useful in the prophylaxis and/or treatment of cell proliferative diseases, infectious diseases, pain, asthma, diabetes, neurodegenerative diseases, cardiovascular diseases and inflammation. The present inventors have previously found that Otviciclib and its derivatives are potent kinase inhibitors and are particularly selective for a subset of cyclin dependent kinases (CDKs), as shown in WO 2018/099952 A1. Protein kinases play important roles in regulating most cellular functions - proliferation/cell cycle, cell metabolism, survival/apoptosis, DNA damage repair, cell motility, response to the microenvironment and inflammatory response. Misregulation of protein kinase function has been found to be associated with oncogenes.

A synthesis and biological activity of certain 3,4-disubstituted pyrazolo[3,4-d]pyrimidine-4-one nucleosides is disclosed in Journal of Medicinal Chemistry, 1984, vol. 27, 9, pp. 1 1 19-1 127. A synthesis of pyrazolo[1,5-a]pyrimidine derivatives has been published in the Journal of the Chinese Chemical Society (Taipei, Taiwan), 2009, vol. 56, 5, 1064-1071.

WO 2018/099952 A1 discloses a synthesis of Otviciclib and derivatives which was successfully used in the preparation of mg amounts. However, in particular when trying to perform this synthesis at a larger scale, low overall yields were obtained. Furthermore, the scale-up is significantly hampered by the required chromatographic purification steps. There has thus been a need for an improved synthesis which allows higher overall yields, especially when performing the synthesis at a multigram or kilogram scale, and avoids chromatographic purifications which is often tedious and involves undesirable solvents.

The objection of the present invention is thus the provision of a process for the preparation of Otviciclib and its derivatives with improved overall yields especially when performing the synthesis at a multigram or kilogram scale while avoiding chromatographic purifications.

The present inventors have surprisingly found that the objects can be achieve by a synthesis as outlined in the claims. This synthesis is a convergent synthesis which avoids chromatographic purifications and provides the desired compounds in high yield.

The new synthesis assembles the final drug in one step (yield of 60-70%) from two independently synthesized building blocks - side chain as well as pyrazole building block. By using the latter component HMPT formation can be avoided. Furthermore, the new synthesis summarizes the critical first three steps of the previous synthesis in a one-pot process. In addition, the present inventors furthermore developed a new synthesis for the underlying non-commercial hydrazine for the production of the pyrazole building block, for which the prior art methods were not suitable at the multigram or kilogram scale.

The process of the present invention preferably contains a step of preparing a compound of formula (VIII) 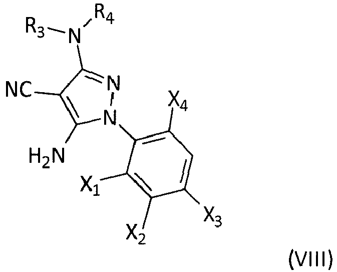 wherein
- X₁: is hydrogen, halogen or C₁₋₆-alkoxy,
- X₂: is hydrogen or halogen,
- X₃: is hydrogen, SO₂NH₂, CONH₂ or COOH,
- X₄: is hydrogen, halogen or C₁₋₆-alkoxy,
- R₃ and R₄: are independently selected from hydrogen, C₁₋₆-alkyl and C₁₋₆-haloalkyl, or a salt thereof,
by a series of reaction steps comprising
the step (t) of reacting propanedinitrile with a compound of formula (XII) 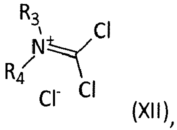 preferably in a solvent containing dichloromethane and diisopropyl ethylamine, to form a compound of formula (X) 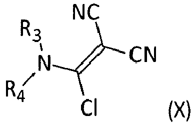 wherein R₃ and R₄ are as defined for formula (VIII),
or a salt thereof;
and step (u) of reacting the compound of formula (X) or the salt thereof with a compound of formula (IX) 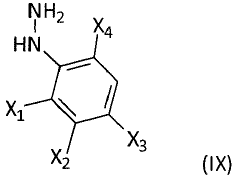 wherein X₁, X₂, X₃ and X₄ are as defined for formula (VIII),
or a salt thereof,
to obtain the compound of formula (VIII) or the salt thereof.

Steps (t) and (u) are preferably performed as a one-pot reaction. Thus, step (t) is conducted and the product of step (t) is directly reacted further by reacting it with the compound of formula (IX) without isolating the compound of formula (X).

By this reaction, the yield of the preparation of the intermediate compound of formula (IX) can be improved about 2-fold as compared to the corresponding reaction reported in WO 2018/099952 A1.

Furthermore, the convergent synthesis of the present invention is based on the finding that compounds of formula (I) 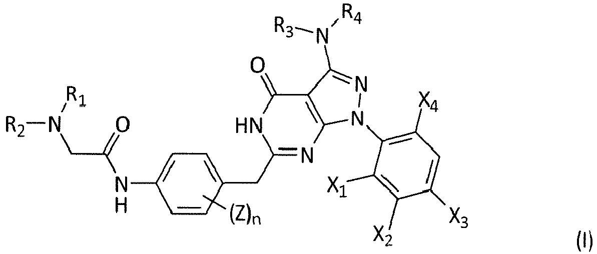 or a salt thereof
can be assembled by a final step of reacting a compound of 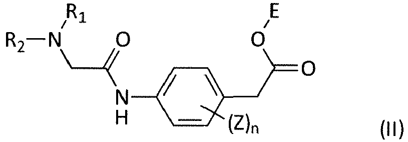 or a salt thereof, with a compound of formula (VII) 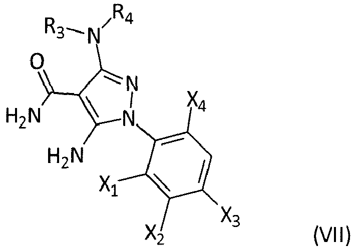 or a salt thereof.

In the compounds discussed herein,
- X₁: is hydrogen, halogen or C₁₋₆-alkoxy,
- X₂: is hydrogen or halogen,
- X₃: is hydrogen, SO₂NH₂, CONH₂ or COOH,
- X₄: is hydrogen, halogen or C₁₋₆-alkoxy,
- R₁ and R₂: are independently selected from hydrogen, C₁₋₆-alkyl and C₁₋₆-acyl, or R₁ and R₂ form, together with the nitrogen atom to which both are attached, a 4 to 8-membered aliphatic heterocycle which contains a total of 1 to 3 heteroatoms selected from N, O and S in the ring, and which is optionally substituted with one or more groups independently selected from halogen, C₁₋₆-alkyl, C₁₋₆-haloalkyl and C₁₋₆-acyl,
- R₃ and R₄: are independently selected from hydrogen, C₁₋₆-alkyl and C₁₋₆-haloalkyl, each Z is independently selected from halogen, CN, C₁₋₆-alkyl, C₁₋₆-alkoxy and C₁₋₆-acyl, and
- n: is an integer from 0 to 4.

### Figures

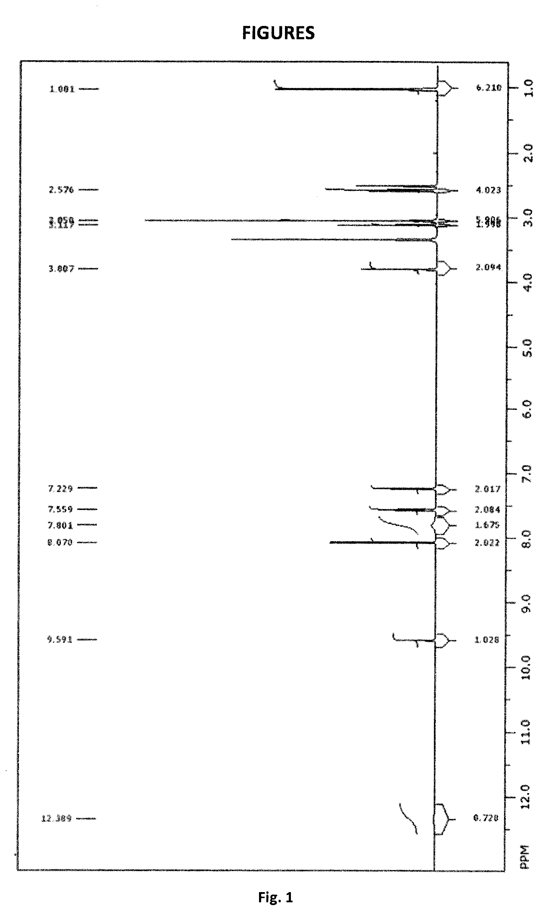

- **Fig. 1**: 700 MHz ¹H-NMR of synthesized Otviciclib (free base)

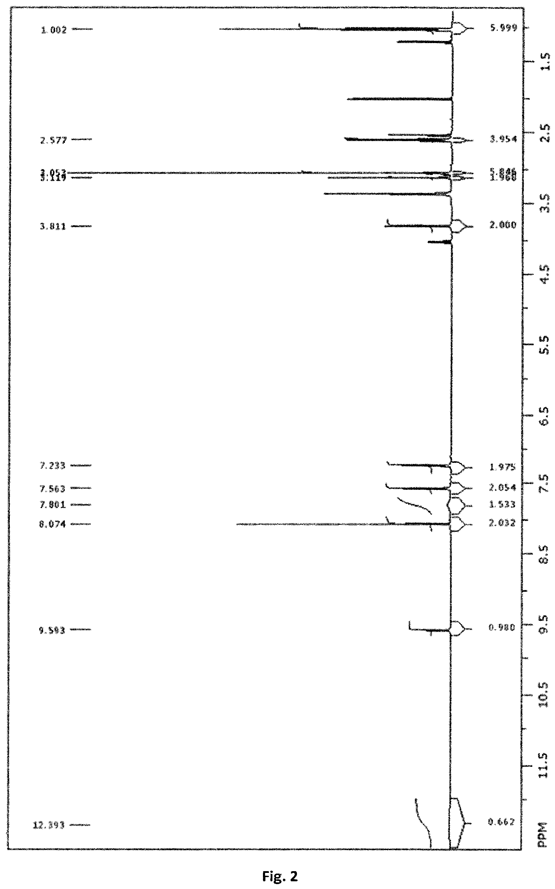

- **Fig. 2**: 600 MHz ¹H-NMR-spectrum (containing trace amounts of ethyl acetate) of Otviciclib (free base) 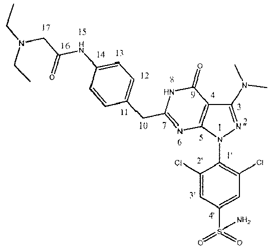

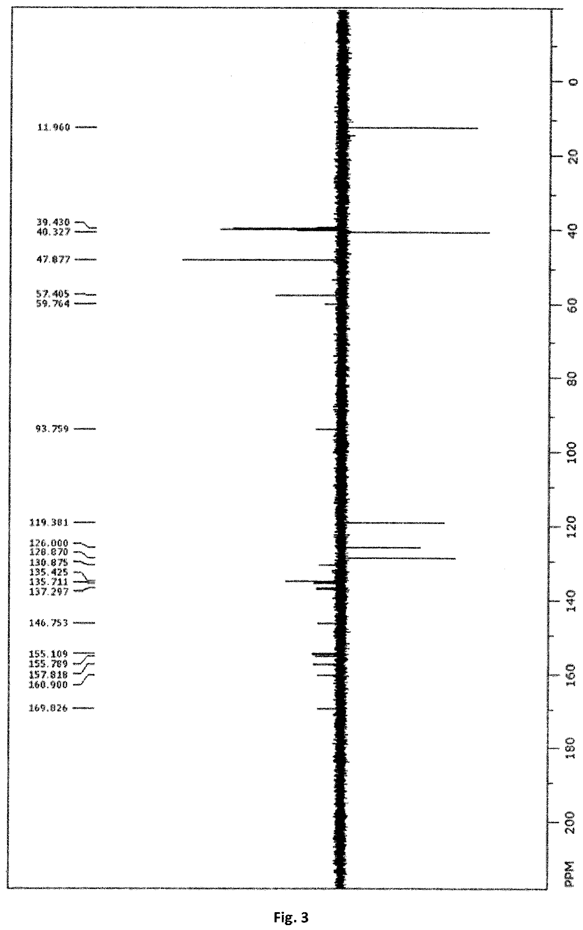

- **Fig. 3**: 151 MHz ¹³C-NMR-spectrum (containing trace amounts of ethyl acetate) of Otviciclib (free base)

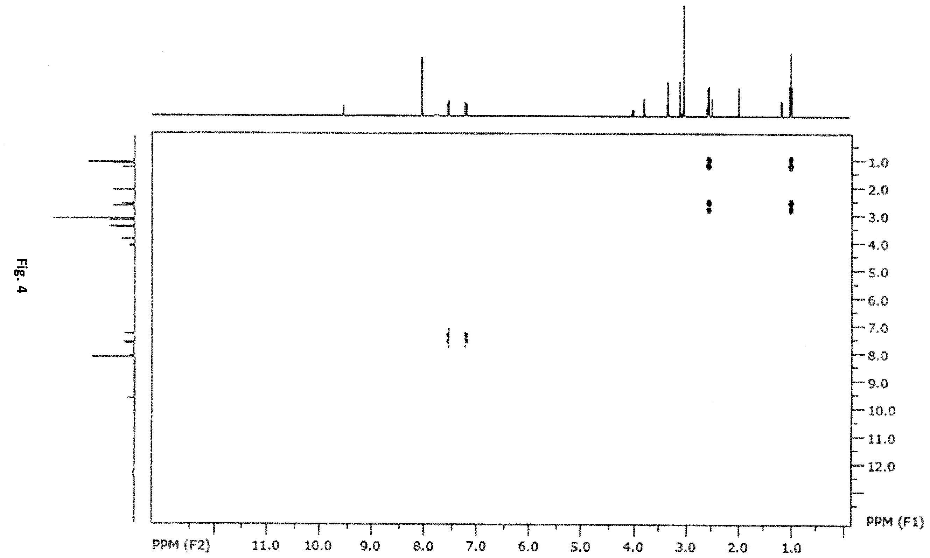

- **Fig. 4**: COSY (homonuclear correlation spectroscopy) 2D-NMR correlation data (containing trace amounts of ethyl acetate) of Otviciclib (free base); showing spins being coupled to each other

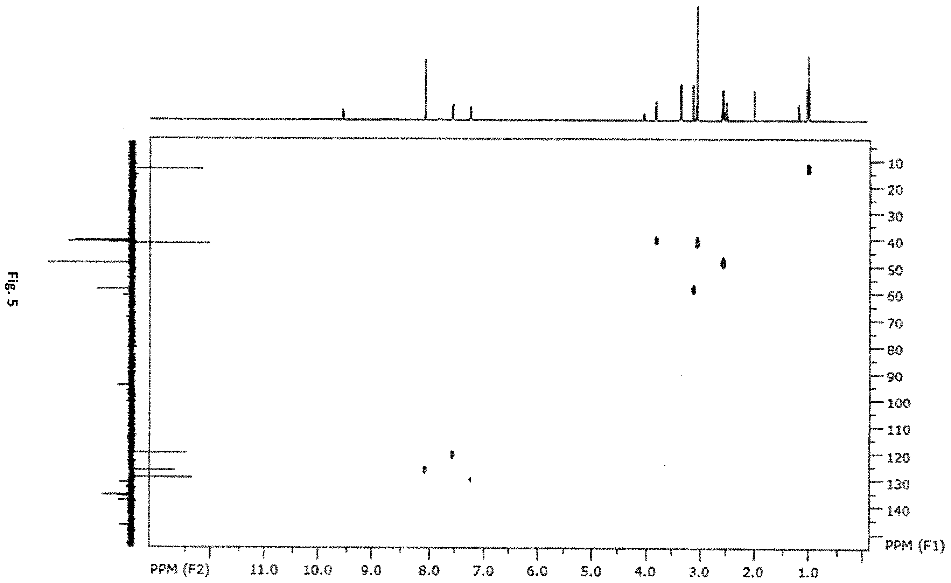

- **Fig. 5**: HSQC (heteronuclear single-quantum correlation spectroscopy) 2D-NMR correlation data (before complete removal of ethyl acetate) of Otviciclib (free base); showing correlations between nuclei of two different types (C and H) which are separated by one bond

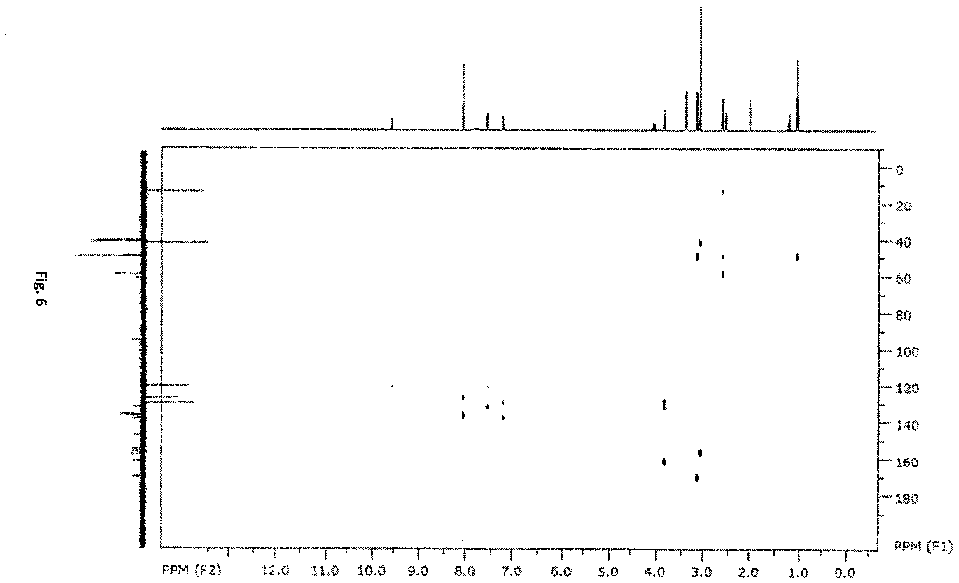

- **Fig. 6**: HMBC (heteronuclear multiple-bond correlation spectroscopy) 2D-NMR correlation data (before complete removal of ethyl acetate) of Otviciclib (free base); showing heteronuclear correlations over about 2 to 4 bonds

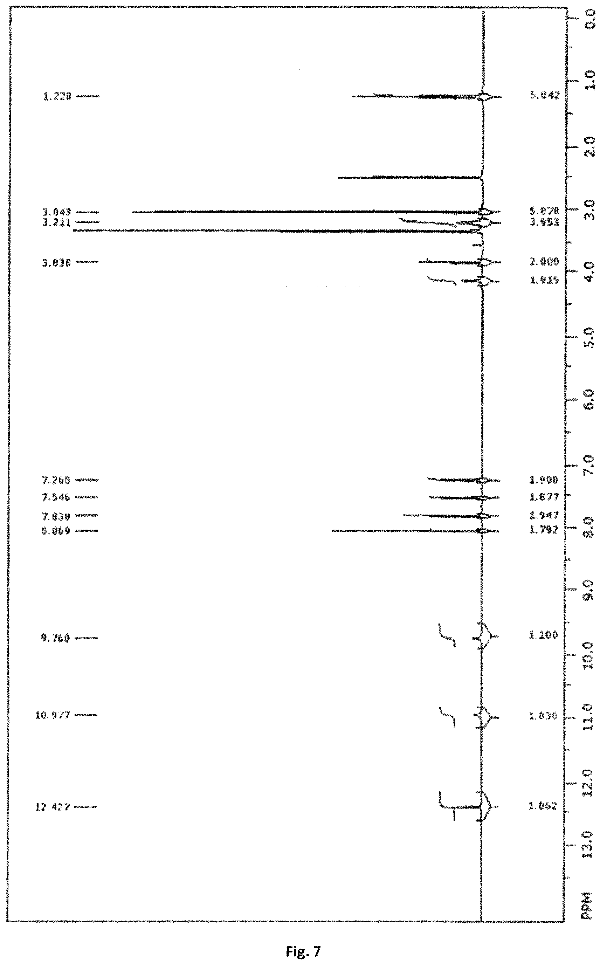

- **Fig. 7**: 600 MHz ¹H-NMR-spectrum of Otviciclib hydrochloride 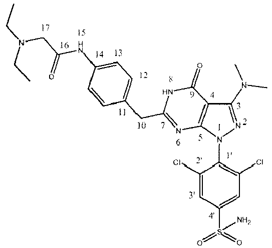

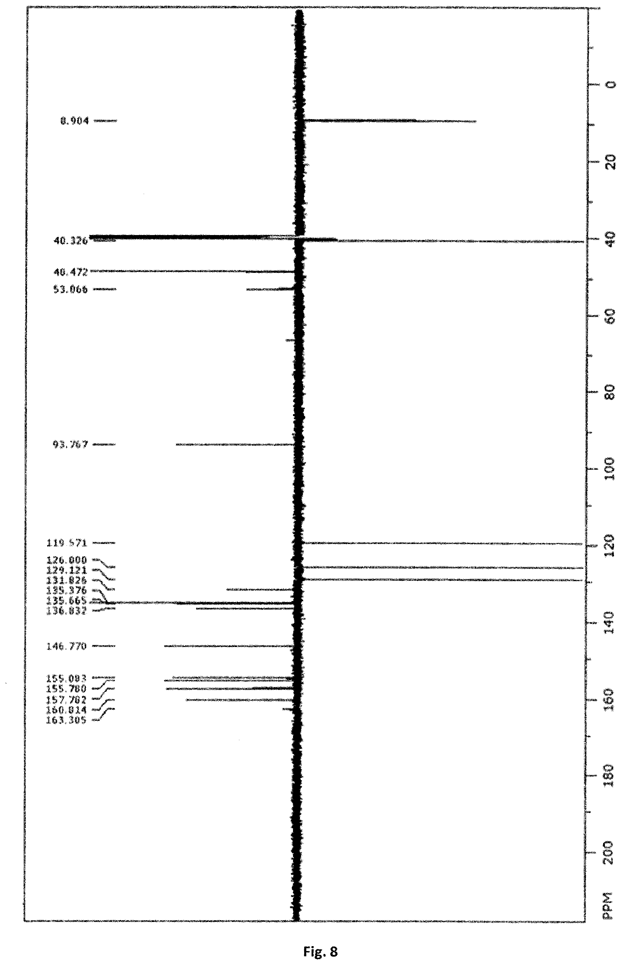

- **Fig. 8**: 151 MHz ¹³C-NMR-spectrum of Otviciclib hydrochloride

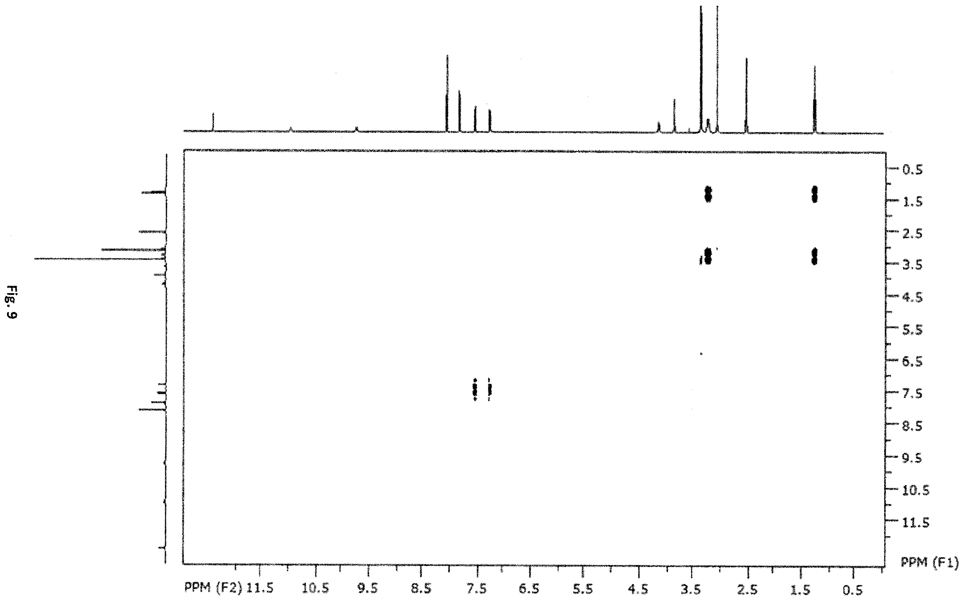

- **Fig. 9**: COSY (homonuclear correlation spectroscopy) 2D-NMR correlation data of Otviciclib hydrochloride; showing spins being coupled to each other

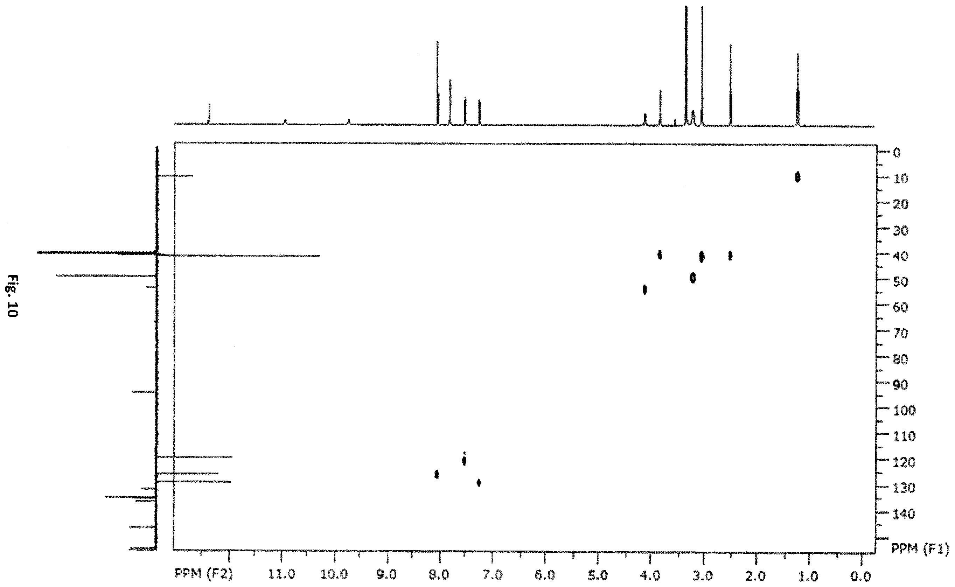

- **Fig. 10**: HSQC (heteronuclear single-quantum correlation spectroscopy) 2D-NMR correlation data of Otviciclib hydrochloride; showing correlations between nuclei of two different types (C and H) which are separated by one bond

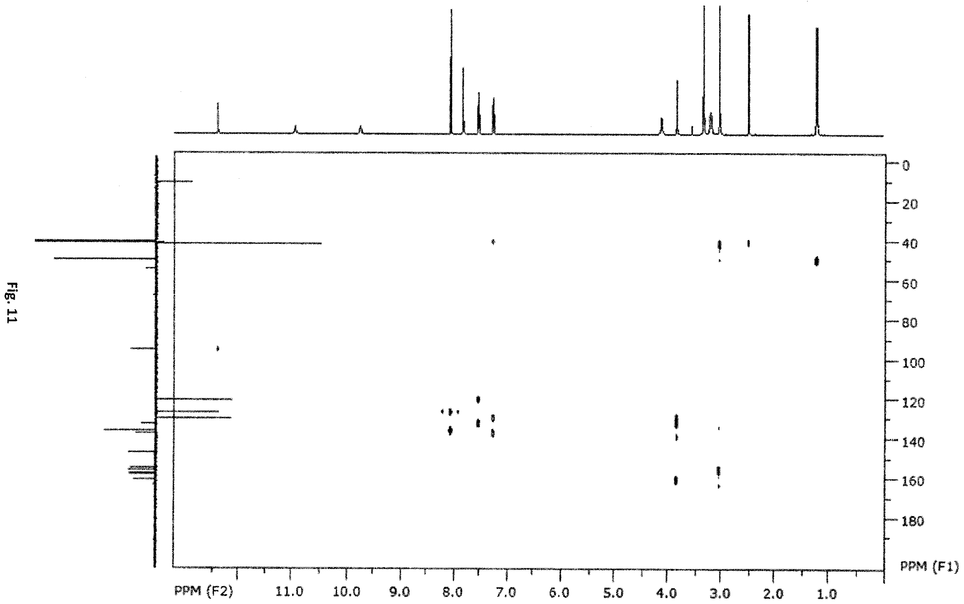

- **Fig. 11**: HMBC (heteronuclear multiple-bond correlation spectroscopy) 2D-NMR correlation data of Otviciclib hydrochloride; showing heteronuclear correlations over about 2 to 4 bonds

### Detailed description

### The method of production of a compound of formula (I)

In a first aspect, the present invention relates to method of production of a compound represented by the following formula (I) 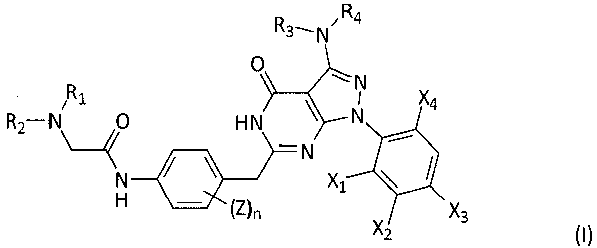 or a salt thereof,
wherein
- X₁: is hydrogen, halogen or C₁₋₆-alkoxy,
- X₂: is hydrogen or halogen,
- X₃: is hydrogen, SO₂NH₂, CONH₂ or COOH,
- X₄: is hydrogen, halogen or C₁₋₆-alkoxy,
- R₁ and R₂: are independently selected from hydrogen, C₁₋₆-alkyl and C₁₋₆-acyl, or R₁ and R₂ form, together with the nitrogen atom to which both are attached, a 4 to 8-membered aliphatic heterocycle which contains a total of 1 to 3 heteroatoms selected from N, O and S in the ring, and which is optionally substituted with one or more groups independently selected from halogen, C₁₋₆-alkyl, C₁₋₆-haloalkyl and C₁₋₆-acyl,
- R₃ and R₄: are independently selected from hydrogen, C₁₋₆-alkyl and C₁₋₆-haloalkyl, each Z is independently selected from halogen, CN, C₁₋₆-alkyl, C₁₋₆-alkoxy and C₁₋₆-acyl,
- n: is an integer from 0 to 4,
wherein the method comprises step (a) of reacting a compound of formula (II) 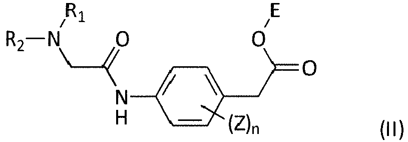 or a salt thereof,
wherein Z, n, R₁ and R₂ as defined for formula (I), and E is C₁₋₆-alkyl,
with a compound of formula (VII) 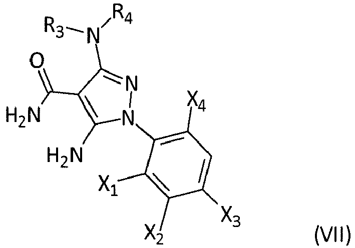
or a salt thereof,
wherein X₁, X₂, X₃, X₄, R₃ and R₄ as defined for formula (I),
to obtain the compound of formula (I) or the salt thereof.

As can be seen from the above, the step of preparing the compound of formula (I) is a step in which two parts of the target compound, each being fully developed, are assembled to obtain the compound of formula (I). A preferred example of the compound of formula (I) is Otviciclib (N-{4-[1-(2,6-Dichloro-4-sulfamoyl-phenyl)-3-dimethylamino-4-oxo-4,5-dihydro-1H-pyrazolo[3,4-d]pyrimidin-6-ylmethyl]-phenyl}-2-diethylamino-acetamide) or a salt thereof. Due to this final assembly step outlined above, the synthesis is a convergent synthesis and can avoid chromatographic purifications and furthermore provide the desired compounds of formula (I) in high yield.

In the formulae described herein, X₁ is preferably F, Cl, or MeO; more preferably F or Cl; even more preferably Cl.

In the formulae described herein, X₂ is preferably hydrogen or F; more preferably hydrogen.

In the formulae described herein, X₃ is preferably hydrogen, SO₂NH₂ or CONH₂; more preferably SO₂NH₂, or CONH₂; even more preferably SO₂NH₂.

In the formulae described herein, X₄ is preferably F, Cl, or MeO; more preferably F or Cl; even more preferably Cl.

It is preferred that R₁ and R₂ are independently selected from C₁₋₆-alkyl; preferably C₁₋₃-alkyl; more preferably methyl, ethyl or 2-propyl; even more preferably ethyl.

Alternatively, it is preferred that R₁ and R₂ form, together with the nitrogen atom to which both are attached, a 5 or 6-membered aliphatic heterocycle which contains a total of 1 to 3 heteroatoms selected from N and O in the ring, and which is optionally substituted with one or more groups independently selected from halogen and C₁₋₆-alkyl. More preferably, R₁ and R₂ form, together with the nitrogen atom to which both are attached, a 6-membered aliphatic heterocycle which contains a total of 1 or 2 heteroatoms selected from N and O in the ring, and which is optionally substituted with one or more independently selected from C₁₋₆-alkyl. Even more preferably, R₁ and R₂ form, together with the nitrogen atom to which both are attached, a piperidine, piperazine or morpholine ring, and each of which is optionally substituted with one or more independently selected from C₁₋₆-alkyl.

R₃ and R₄ are preferably independently selected from C₁₋₆-alkyl and C₁₋₆-haloalkyl; more preferably C₁₋₃-alkyl or C₁₋₃-haloalkyl; even more preferably C₁₋₃-alkyl; still more preferably methyl or ethyl; most preferably methyl.

In a very preferred example of the compound of formula (I) and its method of preparation, X₁ and X₄ are both chloro, X₂ is hydrogen, X₃ is SO₂NH₂, R₁ and R₂ are both ethyl, R₃ and R₄ are both methyl and n is 0.

Preferably, each Z is independently selected from halogen, CN, C₁₋₃-alkyl, C₁₋₃-alkoxy and C₁₋₃-acyl. More preferably, each Z is independently selected from halogen, CN, C₁₋₃-alkyl and C₁₋₃-alkoxy. Even more preferably, each Z is independently selected from halogen, CN and C₁₋₃-alkyl. Still more preferably, each Z is independently selected from halogen, CN and methyl. Most preferably, each Z is independently selected from halogen.

Preferably, n is an integer from 0 to 3, more preferably from 0 to 2. Even more preferably n is 0 or 1. Still more preferably n is 0.

### Step (a)

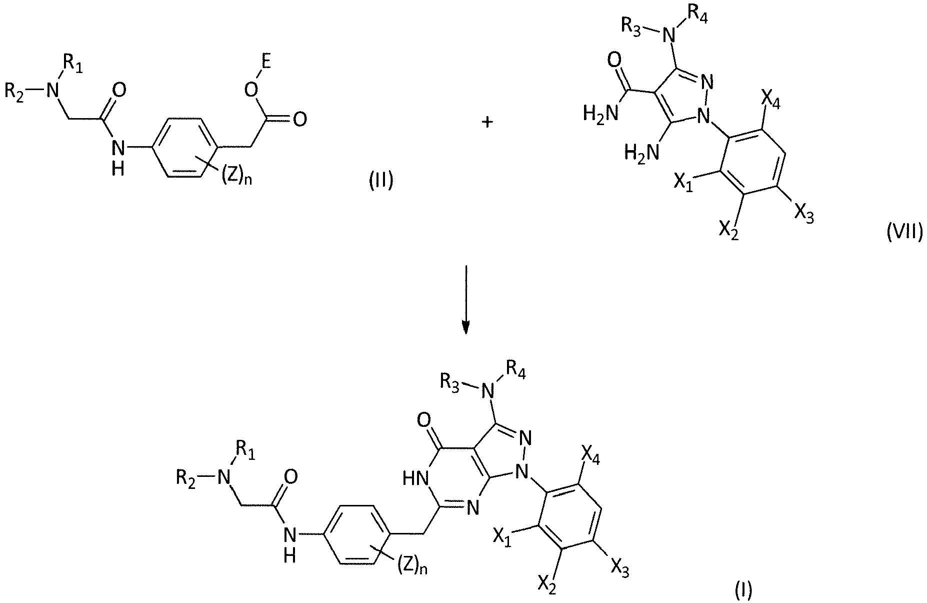

In step (a), compounds (II) and (VII) are preferably reacted in the presence of a base in an aprotic solvent.

It is preferred that a base is added to a mixture of compounds (II) and (VII) in dimethylformamide. More preferably, compounds (II) and (VII) are dissolved in the aprotic solvent and then the base is added.

The addition of the base is preferably conducted while the mixture of compounds (II) and (VII) in the aprotic solvent is cooled, preferably with an ice bath, and/or kept under a protective atmosphere.

The aprotic solvent is preferably an amide of a C₁₋₆ alkanoic acid with a di(C₁₋₆ alkyl)amine, more preferably the aprotic solvent is dimethylformamide.

The base is preferably an alcoholate, more preferably a C₁₋₆ alkanolate, wherein the counter ion is preferably an alkali metal cation (preferably Na⁺ or K⁺). More preferably the base is potassium tert-butoxide.

After the addition of the base, the reaction mixture is preferably heated to a temperature in the range of from 20 to 40°C and preferably allowed to further react for a period of 2 to 24 hours.

The reaction of compounds (II) and (VII) is preferably conducted at a temperature of 0 to 80°C, preferably 20 to 60°C, for 1 to 48 hours, preferably 1 to 4 hours.

For work-up, the reaction mixture is preferably combined with (more preferably added to a mixture of) aqueous sodium chloride solution (such as brine) and an organic solvent (such as ethyl acetate). An acid, such as acetic acid, or a base, such as sodium hydrogen carbonate, may furthermore be added along with the aqueous sodium chloride solution to reach a pH of 7 to 9, preferably about 8.

The purification of compound (I) is not particularly limited. For example, it is possible to purify compound (I) on silica using a mixture of ethyl acetate and methanol (typically 5% methanol in ethyl acetate) as the mobile phase. However, in order to avoid chromatographic purification steps, it is preferably purified by recrystallization.

Generally, the compound of formula (I) can be obtained as a crystalline product during evaporation of the organic solvent used in the work-up (i.e. preferably ethyl acetate).

The present inventors found that particularly high yield and purity can be achieved when compound (I) is purified by recrystallisation from chloroform.

### Step (b)

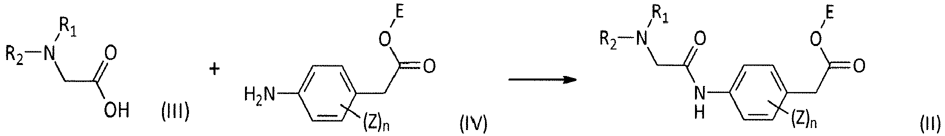

The compound of formula (II) or salt thereof is preferably obtained by step (b) of reacting a compound of formula (III) 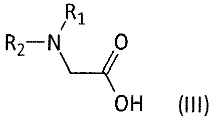
wherein R₁ and R₂ are as defined for formula (I),
or a salt thereof,
with a compound of formula (IV) 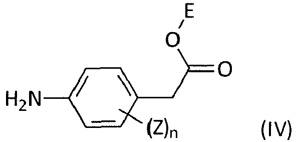
wherein E, Z and n are as defined for formula (II),
or a salt thereof.

The reaction of the compound of formula (III) with the compound of formula (IV) is preferably conducted in the presence of a coupling agent such as are usually used for peptide coupling reactions. These are known to the skilled person and are, e.g., illustrated in Tetrahedron 60 (2004), pages 2447 to 2467. Preferred examples therefore include carbodiimides, such as dicyclohexylcarbodiimide (DCC), diisopropylcarbodiimide (DIC), and ethyl-(*N'*,*N'-*dimethylamino)propylcarbodiimide hydrochloride. Further examples include (benzotriazol-1-yloxy)tris(dimethylamino)phosphonium hexafluorophosphate (BOP), (benzotriazol-1-yloxy)tripyrrolidinophosphonium hexafluorophosphate, (7-azabenzotriazol-1-yloxy)tripyrrolidinophosphonium hexafluorophosphate (PyAOP), bromotripyrrolidinophosphonium hexafluorophosphate, BOP-Cl, O-(benzotriazol-1-yl)-*N*,*N*,*N*',*N*'-tetramethyluronium hexafluorophosphate (HBTU), O-(benzotriazol-1-yl)-*N*,*N*,*N*',*N*'-tetramethyluronium tetrafluoroborate (TBTU), O-(7-azabenzotriazol-1-yl)-*N*,*N*,*N*',*N*'-tetramethyluronium hexafluorophosphate (HATU), O-(7-azabenzotriazol-1-yl)-*N*,*N*,*N*',*N*'-tetramethyluronium tetrafluoroborate (TATU), O-(6-chlorobenzotriazol-1-yl)-*N*,*N*,*N*',*N*'-tetramethyluronium hexafluorophosphate (HCTU), O-(N-succinimidyl)-1,1,3,3-tetramethyl-uronium tetrafluoroborate (TSTU), O-(5-norbornene-2,3-dicarboximido)-*N*,*N*,*N*',*N*'-tetramethyluronium tetrafluoroborate (TNTU), O-(1,2-dihydro-2-oxo-1-pyridyl-*N*,*N*,*N*',*N*'-tetramethyluronium tetrafluoroborate (TPTU), 3-(diethylphosphoryloxy)-1,2,3-benzotriazin-4(3H)-one (DEPBT), and carbonyldiimidazole (CDI). The most preferred coupling agent is dicyclohexylcarbodiimide (DCC).

In addition to the coupling agent, a base is typically used, which is preferably pyridine. Other useful bases include tertiary amines such as diisopropylethylamine and 4-methylmorpholine, and substituted pyridines such as collidine, 2,3,5,6-tetramethylpyridine, and 2,6-di-*tert*-butyl-4-(dimethylamino)pyridine.

In addition to the coupling agent and the base, it is preferred to use a catalyst, which is preferably 4-dimethylamino-pyridine (DMAP).

The reaction of the compound of formula (III) with the compound of formula (IV) is preferably conducted in a solvent comprising dimethylformamide and/or ethyl acetate, preferably a mixture of dimethylformamide and ethyl acetate, more preferably a 1:1 (by volume) mixture of dimethylformamide and ethyl acetate.

It is preferred that the compound of formula (III), the compound of formula (IV), the base and the optional catalyst are mixed with the solvent and then the coupling agent is added in portions.

The reaction mixture is thereafter preferably left to react for a duration in the range of from 2 to 48 hours, preferably from 8 to 24 hours.

Thereafter, the reaction mixture is typically quenched with water, filtered, and then partitioned between toluene and half-saturated brine.

After evaporation of the solvent(s), the residue is preferably dissolved in a mixture of n-pentane and diethyl ether, then filtered through neutral aluminum oxide and the solvent(s) again removed.

### Step (c)

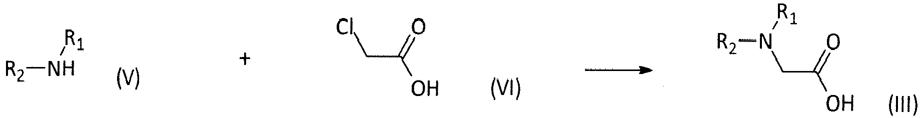

The compound of formula (III) or salt thereof if preferably obtained by step (c) of reacting a compound of formula (V) 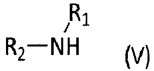
wherein R₁ and R₂ are as defined for formula (I),
or a salt thereof,
with a compound of formula (VI) 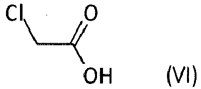
or a salt thereof.

It is preferred that the compound of formula (VI) be mixed with a polar solvent (preferably water) and a base, such as sodium carbonate, be added. This mixture is then, after gas evolution has ceased, typically added dropwise to the compound of formula (V) while cooling, preferably with an ice bath. After the mixture has been added, the reaction is preferably allowed to react, first at a temperature in the range of from 10 to 30°C, preferably from 15 to 25°C, thereafter preferably at a temperature in the range of from 30 to 60°C (such as 45 to 55°C) for preferably 2 to 24 hours, more preferably 4 to 12 hours.

For work-up, the solvent(s) is/are preferably removed and the residue treated with a mixture of isopropanol, ethyl acetate and diethyl ether to remove sodium chloride by filtration. After again removing the solvents, the residue is preferably (in order to obtain an HCl salt of the compound of formula (III)) dissolved in methanol and treated with HCI gas or a solution of HCI gas in methanol, followed by removal of the solvent by evaporation.

### Step (d)

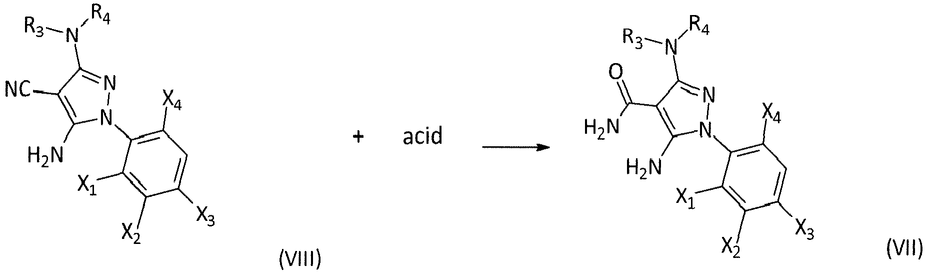

The compound of formula (VII) or salt thereof if preferably obtained by step (d) of reacting a compound of formula (VIII) 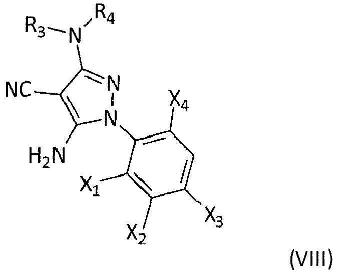 wherein X₁, X₂, X₃, X₄, R₃ and R₄ are as defined for formula (I), or a salt thereof with an acid.

In a preferred step (d), concentrated sulfuric acid as the acid is added to the compound of formula (VIII), preferably followed by the addition of water, and wherein the reaction is preferably conducted at a temperature of from 0 to 50°C for a duration of 2 to 50 hours, whereafter the reaction mixture is preferably quenched with an aqueous sodium hydroxide solution.

The compound of formula (VIII) is preferably combined with the acid at a temperature in the range of from 10 to 30°C, preferably from 15 to 25°C. It is preferred that the acid be added to the compound of formula (VIII).

The acid is preferably a concentrated inorganic acid, more preferably concentrated sulfuric acid. In this context, the concentrated sulfuric acid has preferably a concentration of at least 90 wt.%.

The ratio of concentrated sulfuric acid to the compound of formula (VIII) is preferably in the range of from 500 to 2000 ml/mol, more preferably in the range of from 1000 to 1500 ml/mol.

It is preferred that, after the compound of formula (VIII) has been combined with the acid, e.g. 1 to 10 hours thereafter, water be added. The amount water to be added is preferably in the range of 1 to 20% (more preferably 2 to 10%, such as 5%) relative to the amount of the acid added (ml water/ml acid).

The reaction mixture is thereafter (with or without addition of the water) preferably left to react for a duration in the range of from 2 to 48 hours, preferably from 8 to 24 hours.

Thereafter, an additional amount of acid can be added (typically in cases where the hydrolysis is not yet complete, preferably the same amount as specified above) and the reaction is left to react for a further duration in the range of from 2 to 48 hours, preferably from 8 to 24 hours.

For work-up, the reaction mixture is preferably combined with (preferably added to a mixture of) aqueous alkali hydroxide solution (such as saturated sodium hydroxide solution) and an organic solvent (such as ethyl acetate), while the temperature is preferably controlled with ice cooling. The amount of alkali hydroxide solution is preferably chosen so as to arrive at a final pH in the range of from 2 to 6, preferably from 3 to 5. The alkali hydroxide solution is preferably added batch-wise (preferably under cooling, e.g. with an ice bath), while checking the pH. The pH of the mixture is then preferably adjusted to a range of from 7 to 9, preferably 7.5 to 8.5 (typically using sodium hydrogen carbonate).

After extraction (typically with ethyl acetate) and removal of the solvent(s), the residue is preferably triturated with diethyl ether and optionally washed with further diethyl ether.

### Step (e)

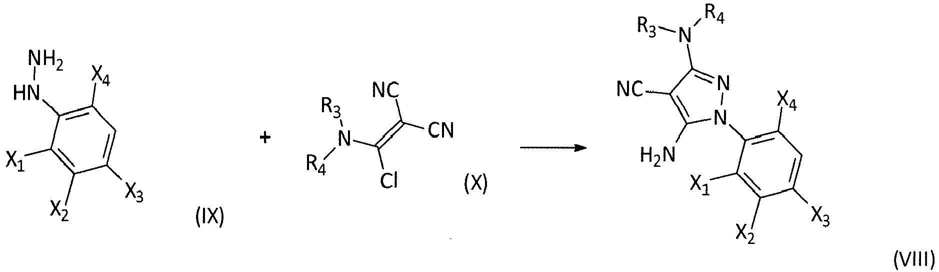

The compound of formula (VIII) or salt thereof if preferably obtained by step (e) of reacting a compound of formula (IX) 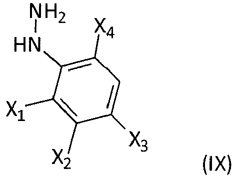
wherein X₁, X₂, X₃ and X₄ are as defined for formula (I),
or a salt thereof,
with a compound of formula (X) 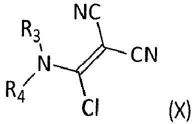
wherein R₃ and R₄ are as defined for formula (I),
or a salt thereof.

In step (e), the compound of formula (X) is believed to be formed as an intermediate by the reaction of propanedinitrile with a compound of formula (XII)

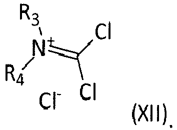

However, the present inventors found that the isolation of the compound of formula (X) significantly reduces the overall yield, presumably because the measures which have to be taken for isolating it led to its partial decomposition. Accordingly, in the present invention, it is preferred that the compound of formula (X) not be isolated but formed in situ by the reaction of propanedinitrile with a compound of formula (XII)

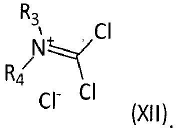

Thus, step (e) may also be expressed as a step of reacting propanedinitrile with a compound of formula (XII) 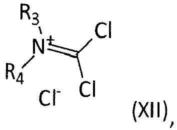 wherein R₃ and R₄ are as defined for formula (I), followed by a reaction of the resulting with a compound of formula (IX) 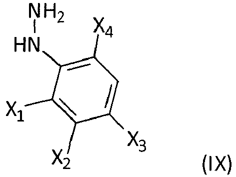
wherein X₁, X₂, X₃ and X₄ are as defined for formula (I),
or a salt thereof.

Such a reaction step, which may also be referred to as a "one-pot reaction" step, has surprisingly been found to lead to an overall yield for this step which is at least about twice as high as in known procedures for the preparation of compounds of formula (VIII).

It is preferred that a mixture of propanedinitrile and an organic solvent be added to a mixture of the compound of formula (XII) and an organic solvent. The organic solvent in both cases is preferably dichloromethane. During the reaction of propanedinitrile and the compound of formula (XII), the temperature of the reaction mixture is preferably kept in a range of from 0 to 10°C, preferably from 2 to 8°C. Cooling is typically conducted with an ice bath. The ratio of propanedinitrile to the compound of formula (XII) is preferably in a range of 1.5:1 to 1:1.5, more preferably about 1:1.

To this reaction mixture, a base (typically in a molar excess, such as 1.5 to 2.5 times the molar amount of the compound of formula (XII)) is preferably added. The base is preferably a tri(C₁₋₆ alkyl)amine, such as N,N-diisopropylethylamine (DIPEA).

The compound of formula (IX) is then combined with (preferably added to) the resulting reaction mixture. For this, the compound of formula (IX) is typically dissolved in an organic solvent preferably having a boiling point higher than dichloromethane, such as dimethyl formamide.

Thus, the solvent used in step (e) is preferably a solvent comprising dichloromethane and N,N-diisopropylethylamine. The dichloromethane is preferably removed from the reaction mixture after the compound of formula (IX) has been added. This may be accomplished by evaporating the dichloromethane by applying a temperature of 30 to 60°C (such as 45 to 55°C) and reducing the pressure to about 50 to 150 mbar.

The reaction mixture is then kept at a temperature of 30 to 60°C (such as 45 to 55°C) for preferably 2 to 24 hours, more preferably 4 to 12 hours.

For work-up, the reaction mixture is typically diluted with water and the resulting mixture extracted with an organic solvent, such as ethyl acetate. Generally, the compound of formula (VIII) can be obtained as a crystalline product during evaporation of the organic solvent used in the work-up (i.e. preferably ethyl acetate).

### Step (f)

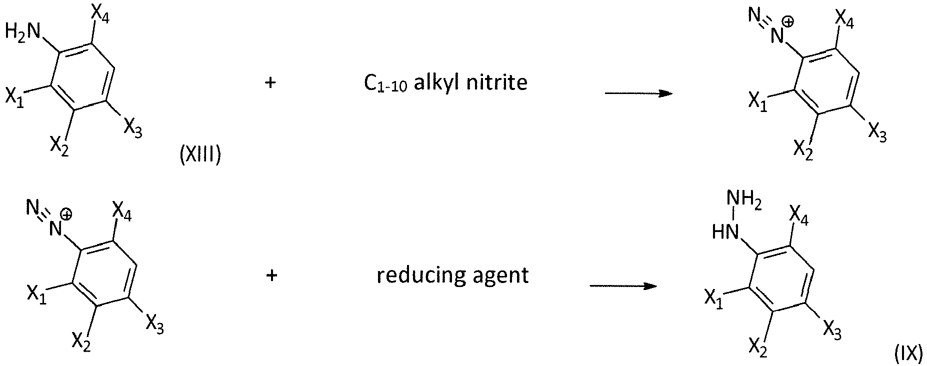

The compound of formula (IX) or salt thereof if preferably obtained by step (f) of reacting a compound of formula (XIII) 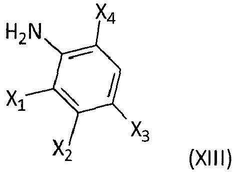
wherein X₁, X₂, X₃ and X₄ are as defined for formula (I),
or a salt thereof,
with a C₁₋₁₀ alkyl nitrite,
followed by reduction.

The C₁₋₁₀ alkyl nitrite is preferably a C₂₋₈ alkyl nitrite, more preferably a C₄₋₆ alkyl nitrite, even more preferably isoamyl nitrite.

In step (f), the compound of formula (XIII) is preferably reacted with the C₁₋₁₀ alkyl nitrite and boron trifluoride diethyl etherate. The C₁₋₁₀ alkyl nitrite is preferably a C₃₋₁₀ alkyl nitrite.

In step (f), the reaction with the C₁₋₁₀ alkyl nitrite and boron trifluoride diethyl etherate is preferably followed by the reduction of the thus formed diazo intermediate with a suitable reducing agent. It is preferred that the diazo intermediate be isolated before the reaction with the reducing agent. The reducing agent is preferably Sn(II)Cl₂ in hydrochloric acid, wherein the hydrochloric acid is preferably concentrated hydrochloric acid, i.e. hydrochloric acid having a concentration of typically at least 20 kg HCl/kg, preferably at least 30 kg HCl/kg, more preferably at least 34 kg HCl/kg.

More preferably, the compound of formula (XIII) is dissolved in an organic solvent, such as tetrahydrofuran, and cooled to a temperature below 0°C, preferably -40 to -1°C, more preferably - 20°C to -2°C, such as -10°C. The boron trifluoride diethyl etherate is thereafter added portion wise. The molar ratio of boron trifluoride diethyl etherate to the compound of formula (XIII) is preferably in the range of from 1.1 to 2, more preferably in the range of from 1.3 to 1.8, such as 1.5. Thereafter, the C₁₋₁₀ alkyl nitrite is typically added while maintaining the cooling, preferably by dropwise addition. The molar ratio of C₁₋₁₀ alkyl nitrite to the compound of formula (XIII) is preferably in the range of from 1.0 to 1.5, more preferably in the range of from 1.1 to 1.3, such as 1.2.

After the addition of the C₁₋₁₀ alkyl nitrite, the reaction mixture is typically allowed to react for a period of 1 to 50 minutes. Diethyl ether is then preferably added, which leads to the crystallization of the corresponding diazonium salt of the compound of formula (XIII) which can be obtained as a solid by filtration. The crystallization of the diazonium salt can be improved by storing at a temperature of 0 to 10°C for a period of e.g. 2 to 24 hours. The solid is then preferably washed with further diethyl ether and the solvent removed in vacuo to isolate the diazonium salt.

The reduction of the diazonium salt is preferably conducted by combining the diazonium salt with concentrated aqueous hydrochloric acid and Sn(II)-chloride. More preferably, the diazonium salt is dissolved in hydrochloric acid at a temperature of about -25 to 0°C, such as - 10 to -5°C and then a solution of Sn(II)-chloride in concentrated aqueous hydrochloric acid is added (preferably dropwise), while continuing the cooling. After the addition of the solution of Sn(II)-chloride in concentrated aqueous hydrochloric acid has been completed, the reaction mixture is typically allowed to further react at a temperature range of preferably -10 to 0°C, e.g. for a period such as 1 minute to 8 hours.

The product of formula 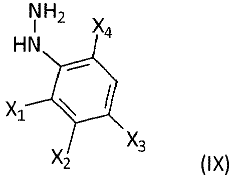 is typically obtained in the form a of a solid (its hydrochloride salt) which begins to form during the addition of the solution of Sn(II)-chloride. This solid can be isolated by filtration and is typically washed with ice water and then dried. For isolation of the compound of formula (IX) from this solid, the solid is preferably mixed with a solvent mixture of ethyl acetate and methanol (typical ratio of ethyl acetate to methanol: about 10:1), and then neutralized with sodium hydrogen carbonate. The compound of formula (IX) is typically extracted with ethyl acetate and isolated by removal of the solvents in vacuo.

### The compound of formula (II)

The present invention also relates to the compound of formula (II)

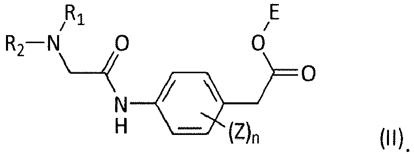

In formula (II), R₁ and R₂ are independently selected from hydrogen, C₁₋₆-alkyl and C₁₋₆-acyl, or R₁ and R₂ form, together with the nitrogen atom to which both are attached, a 4 to 8-membered aliphatic heterocycle which contains a total of 1 to 3 heteroatoms selected from N, O and S in the ring, and which is optionally substituted with one or more groups independently selected from halogen, C₁₋₆-alkyl, C₁₋₆-haloalkyl and C₁₋₆-acyl.

Preferably, R₁ and R₂ in formula (II) are independently selected from C₁₋₆-alkyl; preferably C₁₋₃-alkyl; more preferably methyl, ethyl or 2-propyl; even more preferably ethyl.

Alternatively, R₁ and R₂ in formula (II) form, together with the nitrogen atom to which both are attached, a 5 or 6-membered aliphatic heterocycle which contains a total of 1 to 3 heteroatoms selected from N and O in the ring, and which is optionally substituted with one or more groups independently selected from halogen and C₁₋₆-alkyl. Preferably, R₁ and R₂ form, together with the nitrogen atom to which both are attached, a 6-membered aliphatic heterocycle which contains a total of 1 or 2 heteroatoms selected from N and O in the ring, and which is optionally substituted with one or more independently selected from C₁₋₆-alkyl. More preferably, R₁ and R₂ form, together with the nitrogen atom to which both are attached, a piperidine, piperazine or morpholine ring, and each of which is optionally substituted with one or more independently selected from C₁₋₆-alkyl.

In formula (II), each Z is independently selected from halogen, CN, C₁₋₆-alkyl, C₁₋₆-alkoxy and C₁₋₆-acyl. Preferably, each Z is independently selected from halogen, CN, C₁₋₃-alkyl, C₁₋₃-alkoxy and C₁₋₃-acyl. More preferably, each Z is independently selected from halogen, CN, C₁₋₃-alkyl and C₁₋₃-alkoxy. Even more preferably, each Z is independently selected from halogen, CN and C₁₋₃-alkyl. Still more preferably, each Z is independently selected from halogen, CN and methyl. Most preferably, each Z is independently selected from halogen.

In formula (II), n is an integer from 0 to 4. Preferably, n is an integer from 0 to 3, more preferably from 0 to 2. Even more preferably n is 0 or 1. Still more preferably n is 0.

E in formula (II) is C₁₋₆-alkyl, preferably C₁₋₃-alkyl. More preferably, E is methyl or ethyl, of which methyl is even more preferred.

In even more preferred compounds of formula (II), R₁ and R₂ are both ethyl, n is 0, and E is methyl.

### The method of production of a compound of formula (VIII)

The present invention also relates to a method of production of a compound of formula (VIII) 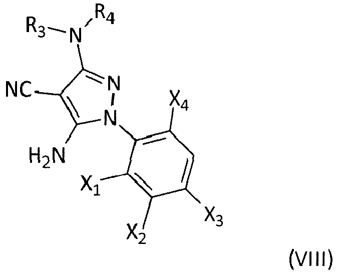 wherein
- X₁ is: hydrogen, halogen or C₁₋₆-alkoxy,
- X₂ is: hydrogen or halogen,
- X₃ is: hydrogen, SO₂NH₂, CONH₂ or COOH,
- X₄ is: hydrogen, halogen or C₁₋₆-alkoxy,

R₃ and R₄ are independently selected from hydrogen, C₁₋₆-alkyl and C₁₋₆-haloalkyl,
or a salt thereof,
comprising the step (t) of reacting propanedinitrile with a compound of formula (XII) 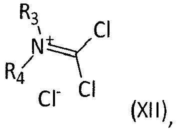 preferably in a solvent containing dichloromethane and diisopropyl ethylamine, to form a compound of formula (X) 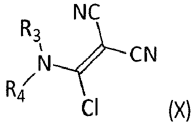
wherein R₃ and R₄ are as defined for formula (VIII),
or a salt thereof;
and step (u) of reacting the compound of formula (X) or the salt thereof with a compound of formula (IX) 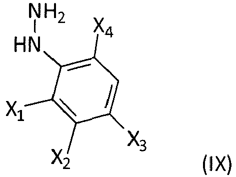 wherein X₁, X₂, X₃ and X₄ are as defined for formula (VIII),
or a salt thereof,
to obtain the compound of formula (VIII) or the salt thereof.

Steps (t) and (u) may together also be referred to as a "one-pot reaction" step because the product of step (t) is preferably not isolated, but directly followed by step (u). This combination has surprisingly been found to lead to an overall yield for this step which is at least about twice as high as in known procedures for the preparation of compounds of formula (VIII).

It is preferred that a mixture of propanedinitrile and an organic solvent be added to a mixture of the compound of formula (XII) and an organic solvent. The organic solvent in both cases is preferably dichloromethane. During the reaction of propanedinitrile and the compound of formula (XII), the temperature of the reaction mixture is preferably kept in a range of from 0 to 10°C, preferably from 2 to 8°C. Cooling is typically conducted with an ice bath. The ratio of propanedinitrile to the compound of formula (XII) is preferably in a range of 1.5:1 to 1:1.5, more preferably about 1:1.

To this reaction mixture, a base (typically in a molar excess, such as 1.5 to 2.5 times the molar amount of the compound of formula (XII)) is preferably added. The base is preferably a tri(C₁₋₆ alkyl)amine, such as *N*,*N*-diisopropylethylamine (DIPEA).

In step (u), the compound of formula (IX) is then combined with (preferably added to) the resulting reaction mixture. For this, the compound of formula (IX) is typically dissolved in an organic solvent preferably having a boiling point (at 1 bar) higher than dichloromethane, such as dimethyl formamide.

Thus, the solvent in step (u) is preferably a solvent comprising dichloromethane (from step (t)) and *N*,*N*-diisopropylethylamine. The dichloromethane is preferably removed from the reaction mixture after the compound of formula (IX) has been added. This may be accomplished by evaporating the dichloromethane by applying a temperature of 30 to 60°C (such as 45 to 55°C) and reducing the pressure to about 50 to 150 mbar.

The reaction mixture is then kept at a temperature of 30 to 60°C (such as 45 to 55°C) for preferably 2 to 24 hours, more preferably 4 to 12 hours.

For work-up, the reaction mixture is typically diluted with water and the resulting mixture extracted with an organic solvent, such as ethyl acetate. Generally, the compound of formula (VIII) can be obtained as a crystalline product during evaporation of the organic solvent used in the work-up (i.e. preferably ethyl acetate).

The meaning of several terms and expression used in the context of the present invention will be explained in further detail in the following.

As used herein, the term "alkyl" refers to a monovalent saturated acyclic (i.e., non-cyclic) hydrocarbon group which may be linear or branched. Accordingly, an "alkyl" group does not comprise any carbon-to-carbon double bond or any carbon-to-carbon triple bond. A "C₁₋₆ alkyl" denotes an alkyl group having 1 to 6 carbon atoms. Preferred exemplary alkyl groups are methyl, ethyl, propyl (e.g., n-propyl or isopropyl), or butyl (e.g., n-butyl, isobutyl, sec-butyl, or tert-butyl). Unless defined otherwise, the term "alkyl" preferably refers to C₁₋₃ alkyl, more preferably to methyl or ethyl, and even more preferably to methyl.

As used herein, the term "alkoxy" refers to an -O-alkyl group, wherein the alkyl moiety comprised in this group is as defined above.

As used herein, the term "acyl" refers to a -C(O)-alkyl group, wherein the alkyl moiety comprised in this group is as defined above. It is to be understood that the carbon of the carbonyl group (C(O)) in "-C(O)-alkyl group" is counted as one of the carbon atoms of the acyl group. Thus, a "C₁₋₆-acyl" group corresponds to a "-C(O)-C₁₋₅-alkyl" group. Unless defined herein otherwise, the term "acyl" preferably refers to C₁₋₄ acyl, more preferably to acetyl or propionyl, and even more preferably to acetyl.

As used herein, the term "halogen" refers to fluoro (-F), chloro (-Cl), bromo (-Br), or iodo (-I), preferably fluoro (-F) or chloro (-Cl).

As used herein, the term "haloalkyl" refers to an alkyl group substituted with one or more (preferably 1 to 6, more preferably 1 to 3) halogen atoms which are selected independently from fluoro, chloro, bromo and iodo, and are preferably all fluoro atoms. It will be understood that the maximum number of halogen atoms is limited by the number of available attachment sites and, thus, depends on the number of carbon atoms comprised in the alkyl moiety of the haloalkyl group. "Haloalkyl" may, e.g., refer to -CF₃, -CHF₂, -CH₂F, -CF₂-CH₃, -CH₂-CF₃, -CH₂-CHF₂, -CH₂-CF₂-CH₃, -CH₂-CF₂-CF₃, or -CH(CF₃)₂. A particularly preferred "haloalkyl" group is -CF₃.

As used herein, the term "4 to 8-membered aliphatic heterocycle which contains a total of 1 to 3 heteroatoms selected from N, O and S in the ring" preferably refers to a monocyclic ring, wherein said monocyclic ring comprises 1 to 3 (such as, e.g., one, two or three) ring heteroatoms independently selected from N, O and S, and the remaining ring atoms are carbon atoms, wherein one or more S ring atoms (if present) and/or one or more N ring atoms may optionally be oxidized, wherein one or more carbon ring atoms may optionally be oxidized (i.e., to form an oxo group). For example, the monocyclic ring may contain one O atom and/or one S atom (which may optionally be oxidized) and/or one, two or three N atoms (which may optionally be oxidized), provided that the total number of heteroatoms in the heterocycle is 1 to 3 and that there is at least one carbon ring atom (which may optionally be oxidized) in the heterocycle. The aliphatic heterocycle is preferably saturated may also be referred to as a heterocycloalkyl. It is to be understood that, when the 4 to 8-membered aliphatic heterocycle is formed by taking R₁ and R₂ together with the nitrogen atom to which both are attached, the 4 to 8-membered aliphatic heterocycle contains at least one nitrogen, i.e. the nitrogen to which both R₁ and R₂ are attached.

As used herein, the terms "optional", "optionally" and "may" denote that the indicated feature may be present but can also be absent. Whenever the term "optional", "optionally" or "may" is used, the present invention specifically relates to both possibilities, i.e., that the corresponding feature is present or, alternatively, that the corresponding feature is absent. For example, the expression "X is optionally substituted with Y" (or "X may be substituted with Y") means that X is either substituted with Y or is unsubstituted. Likewise, if a component of a composition is indicated to be "optional", the invention specifically relates to both possibilities, i.e., that the corresponding component is present (contained in the composition) or that the corresponding component is absent from the composition.

Various groups are referred to as being "optionally substituted" in this specification. Generally, these groups may carry one or more substituents, such as, e.g., one, two, three or four substituents. It will be understood that the maximum number of substituents is limited by the number of attachment sites available on the substituted moiety. Unless defined otherwise, the "optionally substituted" groups referred to in this specification carry preferably not more than two substituents and may, in particular, carry only one substituent. Moreover, unless defined otherwise, it is preferred that the optional substituents are absent, i.e. that the corresponding groups are unsubstituted.

As used herein the terms "preferably", "typically", "such as", "e.g." etc. are to be understood as indicating that the feature, combination of features etc. following these terms is/are preferred (i.e. not mandatory, but believed to lead to improved effects if fulfilled).

As used herein, unless explicitly indicated otherwise or contradicted by context, the terms "a", "an" and "the" are used interchangeably with "one or more" and "at least one". Thus, for example, a composition comprising "a" compound of formulae (I) and (II) can be interpreted as referring to a composition comprising "one or more" compounds of formulae (I) and (II).

As used herein, unless explicitly indicated otherwise or contradicted by context, the term "room temperature" is preferably 20°C.

As used herein, the term "comprising" (or "comprise", "comprises", "contain", "contains", or "containing"), unless explicitly indicated otherwise or contradicted by context, has the meaning of "containing, inter alia", i.e., "containing, among further optional elements,...". In addition thereto, this term also includes the narrower meanings of "consisting essentially of" and "consisting of". For example, the term "A comprising B and C" has the meaning of "A containing, inter alia, B and C", wherein A may contain further optional elements (e.g., "A containing B, C and D" would also be encompassed), but this term also includes the meaning of "A consisting essentially of B and C" and the meaning of "A consisting of B and C" (i.e., no other components than B and C are comprised in A).

The steps of the methods of the present invention are preferably conducted under "protective atmosphere". Protective atmosphere preferably refers to a gas or mixture of gases with a relative humidity (at 20°C and 1 bar) of less than 20%, preferably 10% or less, more preferably 5% or less, even more preferably 2% or less, still more preferably 1% or less, even still more preferably 0.2% or less. The protective atmosphere furthermore preferably contains oxygen at a concentration of (at 20°C and 1 bar) less than 10 vol.-%, preferably 5 mol-% or less, more preferably 1 mol-% or less, even more preferably 0.5 mol-% or less, still more preferably 0.1 mol-% or less, even still more preferably 0.01 mol-% or less. Furthermore, the gas or mixture of gases is preferably one or more selected from nitrogen, argon, carbon dioxide. The gas or mixture of gases is more preferably nitrogen or argon or a mixture thereof.

Moreover, unless indicated otherwise, any reference to an industry standard, a pharmacopeia, or a manufacturer's manual refers to the corresponding latest version that was available at the priority date (i.e., at the earliest filing date) of the present specification.

Furthermore, the compounds provided herein, may exist in the form of different isomers, in particular stereoisomers (including, e.g., geometric isomers (or cis/trans isomers), enantiomers and diastereomers) or tautomers. All such isomers of the compounds provided herein are contemplated as being part of the present invention, either in admixture or in pure or substantially pure form. As for stereoisomers, the invention embraces the isolated optical isomers of the compounds according to the invention as well as any mixtures thereof (including, in particular, racemic mixtures/racemates). The racemates can be resolved by physical methods, such as, e.g., fractional crystallization, separation or crystallization of diastereomeric derivatives, or separation by chiral column chromatography. The individual optical isomers can also be obtained from the racemates via salt formation with an optically active acid followed by crystallization. The present invention further encompasses any tautomers of the compounds provided herein.

In the compounds referred to herein one or more atoms can be replaced by a specific isotope of the corresponding atom. For example, one or more hydrogen atoms (or, e.g., all hydrogen atoms) can be replaced by deuterium atoms (i.e., ²H; also referred to as "D"). Accordingly, also compounds which are enriched in deuterium are encompassed. Naturally occurring hydrogen is an isotopic mixture comprising about 99.98 mol-% hydrogen-1 (¹H) and about 0.0156 mol-% deuterium (²H or D). The content of deuterium in one or more hydrogen positions in the compounds disclosed herein can be increased using deuteration techniques known in the art. For example, a compound can be subjected to an H/D exchange reaction using, e.g., heavy water (D₂O). Further suitable deuteration techniques are described in: Atzrodt J et al., Bioorg Med Chem, 20(18), 5658-5667, 2012; William JS et al., Journal of Labelled Compounds and Radiopharmaceuticals, 53(11-12), 635-644, 2010; or Modvig A et al., J Org Chem, 79, 5861-5868, 2014. The content of deuterium can be determined, e.g., using mass spectrometry or NMR spectroscopy. Unless specifically indicated otherwise, it is preferred that the compounds disclosed herein are not enriched in deuterium. Accordingly, the presence of naturally occurring hydrogen atoms or ¹H hydrogen atoms in the compounds disclosed herein is preferred.

In the compounds referred to herein one or more atoms can be replaced by a positronemitting isotope of the corresponding atom, such as, e.g., ¹⁸F, ¹¹C, ¹³N, ¹⁵O, ⁷⁶Br, ⁷⁷Br, ¹²⁰I and/or ¹²⁴I.

Several compounds referred to herein are referred to as the compounds as such or may also be in the form of a salt, or in the form of salts. Such salts may be formed, e.g., by protonation of an atom carrying an electron lone pair which is susceptible to protonation, such as an amino group, with an inorganic or organic acid, or as a salt of an acid group (such as a carboxylic acid group) with a cation.

Exemplary base addition salts comprise, for example: alkali metal salts such as sodium or potassium salts; alkaline earth metal salts such as calcium or magnesium salts; zinc salts; ammonium salts; aliphatic amine salts such as trimethylamine, triethylamine, dicyclohexylamine, ethanolamine, diethanolamine, triethanolamine, procaine salts, meglumine salts, ethylenediamine salts, or choline salts; aralkyl amine salts such as N,N-dibenzylethylenediamine salts, benzathine salts, benethamine salts; heterocyclic aromatic amine salts such as pyridine salts, picoline salts, quinoline salts or isoquinoline salts; or quaternary ammonium salts such as tetramethylammonium salts, tetraethylammonium salts, benzyltrimethylammonium salts, benzyltriethylammonium salts, benzyltributylammonium salts, methyltrioctylammonium salts or tetrabutylammonium salts.

Exemplary acid addition salts comprise, for example: mineral acid salts such as hydrochloride, hydrobromide, hydroiodide, sulfate salts (such as, e.g., sulfate or hydrogensulfate salts), nitrate salts, phosphate salts (such as, e.g., phosphate, hydrogenphosphate, or dihydrogenphosphate salts), carbonate salts, hydrogencarbonate salts, perchlorate salts, borate salts, or thiocyanate salts; organic acid salts such as acetate, propionate, butyrate, pentanoate, hexanoate, heptanoate, octanoate, cyclopentanepropionate, decanoate, undecanoate, oleate, stearate, lactate, maleate, oxalate, fumarate, tartrate, malate, citrate, succinate, adipate, gluconate, glycolate, nicotinate, benzoate, salicylate, ascorbate, pamoate (embonate), camphorate, glucoheptanoate, or pivalate salts; or sulfonate salts such as methanesulfonate (mesylate), ethanesulfonate (esylate), 2-hydroxyethanesulfonate (isethionate), benzenesulfonate (besylate), p-toluenesulfonate (tosylate), 2-naphthalenesulfonate (napsylate), 3-phenylsulfonate, or camphorsulfonate salts.

It is to be understood that the present invention specifically relates to each and every combination of features and embodiments described herein, including any combination of general and/or preferred features/embodiments. In particular, the invention specifically relates to each combination of meanings (including general and/or preferred meanings) for the various groups, features and variables disclosed herein.

In this specification, a number of documents including patent applications, scientific literature and manufacturers' manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

The invention will be illustrated further by reference to specific experimental examples. It is to be understood that the scope of the present invention is not limited by these examples. As a skilled person will understand based on the present disclosure, the present invention may be performed in many other ways while remaining within the scope of the present invention.

The present invention may be summarized by the following items 1 to 22:
1. A method of production of a compound represented by the following formula (I) 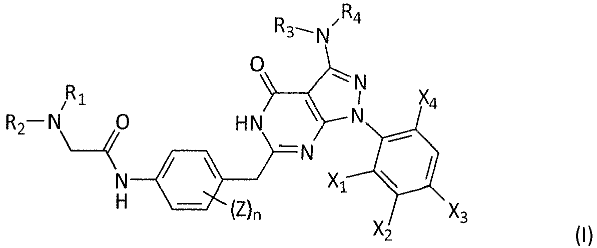 or a salt thereof,
   wherein
   X₁ is hydrogen, halogen or C₁₋₆-alkoxy,
   X₂ is hydrogen or halogen,
   X₃ is hydrogen, SO₂NH₂, CONH₂ or COOH,
   X₄ is hydrogen, halogen or C₁₋₆-alkoxy,
   R₁ and R₂ areindependently selected from hydrogen, C₁₋₆-alkyl and C₁₋₆-acyl,
   or R₁ and R₂ form, together with the nitrogen atom to which both are attached, a 4 to 8-membered aliphatic heterocycle which contains a total of 1 to 3 heteroatoms selected from N, O and S in the ring, and which is optionally substituted with one or more groups independently selected from halogen, C₁₋₆-alkyl, C₁₋₆-haloalkyl and C₁₋₆-acyl,
   R₃ and R₄ areindependently selected from hydrogen, C₁₋₆-alkyl and C₁₋₆-haloalkyl,
   each Z is independently selected from halogen, CN, C₁₋₆-alkyl, C₁₋₆-alkoxy and C₁₋₆-acyl,
   n is an integer from 0 to 4,
   wherein the method comprises step (a) of reacting a compound of formula (II) 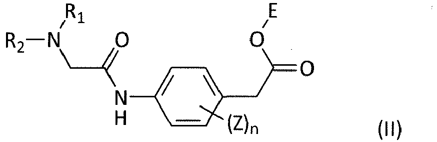
   or a salt thereof,
   wherein Z, n, R₁ and R₂ as defined for formula (I), and E is C₁₋₆-alkyl,
   with a compound of formula (VII) 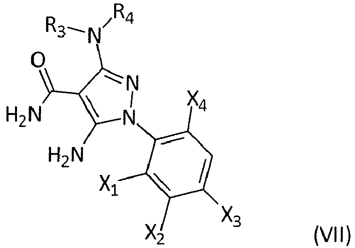
   or a salt thereof,
   wherein X₁, X₂, X₃, X₄, R₃ and R₄ as defined for formula (I),
   to obtain the compound of formula (I) or the salt thereof.
2. The method according to item 1, wherein in step (a), compounds (II) and (VII) are reacted in the presence of a base in an aprotic solvent, preferably wherein a base is added to a mixture of compounds (II) and (VII) in dimethylformamide.
3. The method according to item 2, wherein
   - the base is an alcoholate, preferably potassium tert-butanolate;
   - the reaction of compounds (II) and (VII) is conducted under a protective atmosphere;
   - the reaction of compounds (II) and (VII) is conducted at a temperature of 0 to 80°C, preferably 20 to 60°C, for 1 to 48 hours, preferably 1 to 4 hours, and/or
   - compound (I) is recrystallized from chloroform.
4. The method according to any one of items 1 to 3, wherein the method furthermore comprises the following step (b) of reacting a compound of formula (III) 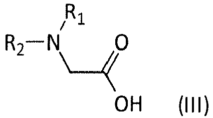
   wherein R₁ and R₂ as defined for formula (I),
   or a salt thereof,
   with a compound of formula (IV) 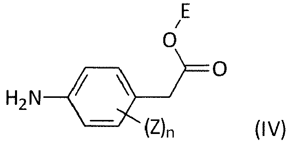
   wherein E, Z and n are as defined for formula (II),
   or a salt thereof,
   to obtain the compound of formula (II) or a salt thereof.
5. The method according to item 4, wherein the method furthermore comprises the following step (c) of reacting a compound of formula (V) 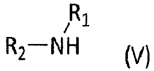
   wherein R₁ and R₂ as defined for formula (I),
   or a salt thereof,
   with a compound of formula (VI) 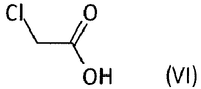
   or a salt thereof,
   to obtain the compound of formula (III) or the salt thereof.
6. The method according to any one of items 1 to 5, wherein the method furthermore comprises the following step (d) of reacting a compound of formula (VIII) 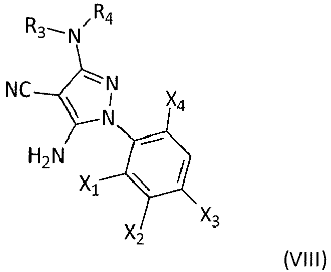
   wherein X₁, X₂, X₃, X₄, R₃ and R₄ are as defined for formula (I),
   or a salt thereof,
   with an acid to obtain the compound of formula (VII) or the salt thereof.
7. The method according to item 6, wherein in step (d), concentrated sulfuric acid as the acid is added to the compound of formula (VIII), preferably followed by the addition of water, and wherein the reaction is preferably conducted at a temperature of from 0 to 50°C for a duration of 2 to 50 hours, whereafter the reaction mixture is preferably quenched with an aqueous sodium hydroxide solution.
8. The method according to items 6 and 7, wherein the method furthermore comprises the following step (e) of reacting a compound of formula (IX) 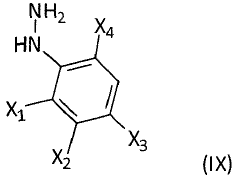
   wherein X₁, X₂, X₃ and X₄ are as defined for formula (I),
   or a salt thereof,
   with a compound of formula (X) 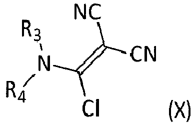
   wherein R₃ and R₄ are as defined for formula (I),
   or a salt thereof
   to obtain the compound of formula (VIII) or the salt thereof.
9. The method according to item 8, wherein in step (e), the compound of formula (X) is formed as an intermediate by the reaction of propanedinitrile with a compound of formula (XII) 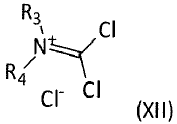 preferably in a solvent containing dichloromethane and diisopropyl ethylamine.
10. The method according to item 8, wherein the method furthermore comprises the following step (f) of reacting a compound of formula (XIII) 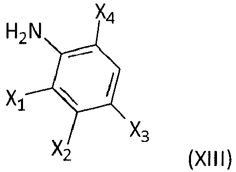
   wherein X₁, X₂, X₃ and X₄ are as defined for formula (I),
   or a salt thereof,
   with a C₁₋₁₀ alkyl nitrite,
   followed by reduction,
   to obtain the compound of formula (IX) or the salt thereof.
11. The method according to item 10, wherein in step (f), the compound of formula (XIII) is reacted with the C₁₋₁₀ alkyl nitrite, which is preferably isoamyl nitrite, and boron trifluoride diethyl etherate, following by the reduction of the thus formed intermediate with Sn(II)Cl₂ in concentrated hydrochloric acid.
12. The method according to any one of the preceding items, wherein
   - X₁ is: F, Cl, or MeO; preferably F or Cl; more preferably Cl;
   - X₂ is: hydrogen, or F; preferably hydrogen;
   - X₃ is: hydrogen, SO₂NH₂, or CONH₂; more preferably SO₂NH₂, or CONH₂; even more preferably SO₂NH₂; and
   - X₄ is: F, Cl, or MeO; preferably F or Cl; more preferably Cl.
13. The method according to any one of the preceding items, wherein R₃ and R₄ are independently selected from C₁₋₆-alkyl and C₁₋₆-haloalkyl; preferably C₁₋₃-alkyl or C₁₋₃-haloalkyl; more preferably C₁₋₃-alkyl; even more preferably methyl or ethyl; still more preferably methyl.
14. The method according to any one of the preceding items, wherein X₁ and X₄ are both chloro, X₂ is hydrogen, X₃ is SO₂NH₂, R₁ and R₂ are both ethyl, R₃ and R₄ are both methyl and n is 0.
15. A compound of formula (II) 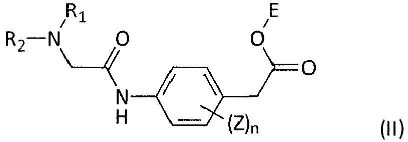
   R₁ and R₂ are independently selected from hydrogen, C₁₋₆-alkyl and C₁₋₆-acyl,
   or R₁ and R₂ form, together with the nitrogen atom to which both are attached, a 4 to 8-membered aliphatic heterocycle which contains a total of 1 to 3 heteroatoms selected from N, O and S in the ring, and which is optionally substituted with one or more groups independently selected from halogen, C₁₋₆-alkyl, C₁₋₆-haloalkyl and C₁₋₆-acyl,
   each Z is independently selected from halogen, CN, C₁₋₆-alkyl, C₁₋₆-alkoxy and C₁₋₆-acyl;
   n is an integer from 0 to 4, and
   E is C₁₋₆-alkyl.
16. The method according to any one of items 1 to 13 or the compound of item 15, wherein each Z is independently selected from halogen, CN, C₁₋₃-alkyl, C₁₋₃-alkoxy and C₁₋₃-acyl; preferably halogen, CN, C₁₋₃-alkyl and C₁₋₃-alkoxy; more preferably halogen, CN and C₁₋₃-alkyl; even more preferably halogen, CN and methyl, still more preferably halogen.
17. The method according to any one of items 1 to 13 and 16 or the compound of item 15 or 16, wherein n is an integer from 0 to 3; preferably from 0 to 2; more preferably 0 or 1; even more preferably 0.
18. The compound according to any one of items 15 to 17, wherein E is C₁₋₃-alkyl, preferably methyl or ethyl, more preferably methyl.
19. The compound according to any one of items 15 to 18, wherein R₁ and R₂ are both ethyl, n is 0, and E is methyl.
20. The method according to any one of items 1 to 13 and 16 to 18 or the compound of any one of item 15 to 18, wherein R₁ and R₂ are independently selected from C₁₋₆-alkyl; preferably C₁₋₃-alkyl; more preferably methyl, ethyl or 2-propyl; even more preferably ethyl.
21. The method according to any one of items 1 to 13 and 16 to 18 or the compound of any one of items 15 to 18, wherein R₁ and R₂ form, together with the nitrogen atom to which both are attached, a 5 or 6-membered aliphatic heterocycle which contains a total of 1 to 3 heteroatoms selected from N and O in the ring, and which is optionally substituted with one or more groups independently selected from halogen and C₁₋₆-alkyl;
   R₁ and R₂ preferably form, together with the nitrogen atom to which both are attached, a 6-membered aliphatic heterocycle which contains a total of 1 or 2 heteroatoms selected from N and O in the ring, and which is optionally substituted with one or more independently selected from C₁₋₆-alkyl;
   R₁ and R₂ more preferably form, together with the nitrogen atom to which both are attached, a piperidine, piperazine or morpholine ring, and each of which is optionally substituted with one or more independently selected from C₁₋₆-alkyl.
22. A method of production of a compound of formula (VIII) 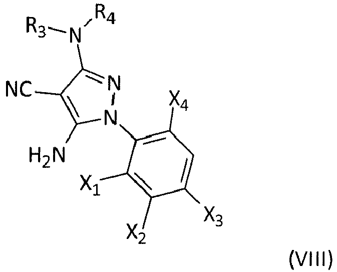 wherein
   - X₁ is: hydrogen, halogen or C₁₋₆-alkoxy,
   - X₂ is: hydrogen or halogen,
   - X₃ is: hydrogen, SO₂NH₂, CONH₂ or COOH,
   - X₄ is: hydrogen, halogen or C₁₋₆-alkoxy,
   - R₃ and R₄: areindependently selected from hydrogen, C₁₋₆-alkyl and C₁₋₆-haloalkyl, or a salt thereof,
comprising the step (t) of reacting propanedinitrile with a compound of formula (XII) 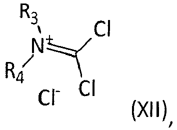 preferably in a solvent containing dichloromethane and diisopropyl ethylamine, to form a compound of formula (X) 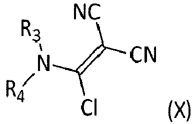
wherein R₃ and R₄ are as defined for formula (VIII),
or a salt thereof;
and step (u) of reacting the compound of formula (X) or the salt thereof with a compound of formula (IX) 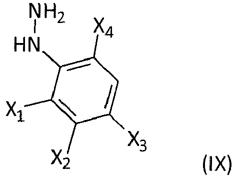
wherein X₁, X₂, X₃ and X₄ are as defined for formula (VIII),
or a salt thereof,
to obtain the compound of formula (VIII) or the salt thereof.

### Examples

### Synthesis procedures for Otviciclib (N-{4-[1-(2,6-Dichloro-4-sulfamoyl-phenyl)-3-dimethylamino-4-oxo-4,5-dihydro-1H-pyrazolo[3,4-d] pyrimidin-6-ylmethyl]-phenyl}-2-diethylamino-acetamide)

### Part A: Synthesis of building blocks:

### 1) Diethylamino-acetic acid hydrochloride (1) (corresponding to step (c) in the general part of the description)

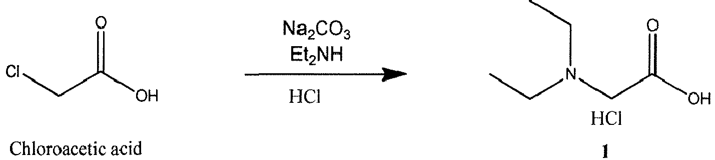

Chloroacetic acid (100 g, 1.06 mol) was dissolved in 250 mL of water. To the solution was added sodium carbonate (56 g, 0.53 mol) in portions. The reaction mixture was stirred until gas evolution ceased. The solution obtained was added dropwise within 15 min under vigorous stirring and ice cooling to diethyl amine (260 mL, 2.52 mol). The ice cooling was removed. The reaction mixture was stirred for one hour at room temperature and kept under stirring at 50°C overnight. The orange-colored solution obtained was liberated from volatiles under reduced pressure. The remaining orange oil was treated twice with a mixture of isopropanol, ethyl acetate and diethyl ether to remove sodium chloride by filtration. The volatiles were removed under reduced pressure. The oily residue obtained was dissolved in dry methanol. Dry HCl-gas was passed through the solution for 5 min. The reaction mixture was stirred for 30 min. Volatile material was completely removed under reduced pressure. Yield: 141.3 g (80 %) of **diethylamino-acetic acid hydrochloride (1)** as creamy residue.

### 2) [4-(2-Diethylamino-acetylamino)-phenyl]-acetic acid methyl ester (3) (corresponding to step (b) in the general part of the description)

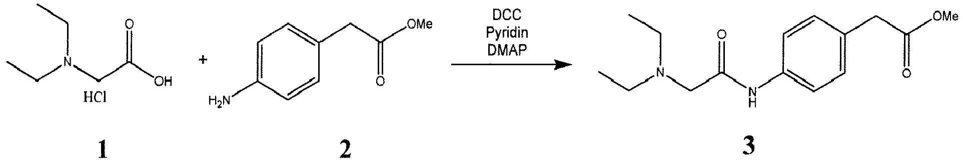

Diethylamino-acetic acid hydrochloride (**1**) (16.8 g, 0.1 mol), (4-amino-phenyl)-acetic acid methyl ester (**2**) (14.14 g, 0.086 mol), 4-dimethylamino-pyridine (DMAP, 100 mg) and pyridine (16.5 mL, 0.2 mol) were dissolved in a mixture of dry DMF (40 mL) and dry ethyl acetate (40 mL). To the solution obtained was added DCC (*N*,*N'*-dicyclohexylcarbodiimide) (20.6 g, 0.1 mol) in portions and stirred at room temperature for 16 hours. TLC (ethyl acetate/petrol ether = 4/1) indicated full conversion to the product. The reaction mixture was diluted with water (50 mL) and stirred for 15 min. Solid material was removed by filtration through a cotton plug. The remaining solution was treated with toluene and half-saturated aqueous sodium chloride solution. The organic phase obtained was consecutively washed with saturated aqueous sodium hydrogen carbonate solution and saturated aqueous sodium chloride solution. The organic phase was dried over magnesium sulfate, filtered and concentrated under reduced pressure. The residual obtained was dissolved in n-pentane and a minimum of diethyl ether. The solution obtained was filtered through neutral aluminum oxide and concentrated under reduced pressure. Finally, the residual red oil was filtered through a cotton plug with n-pentane as eluent. The n-pentane was removed under high vacuum. Yield: 16.8 g (71 %) of **[4-(2-diethylamino-acetylamino)-phenyl]-acetic acid methyl ester (3)** as a red viscous oil.

### 3) 3,5-dichloro-4-hydrazinylbenzenesulfonamide (6)

Synthesis of 6 is described in PCT WO 03/063764 A2 (p 35). This procedure applies a work-up by chromatography. The procedure used herein is described in the following.

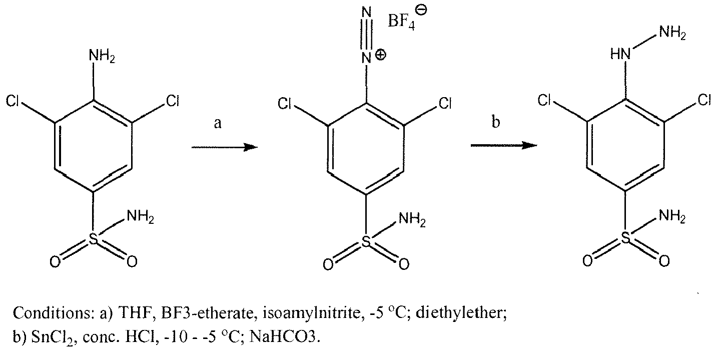

4-Amino-3,5-dichloro-benzenesulfonamide (24.85 g; 103 mmol) was dissolved in 200 mL of THF. The solution obtained was cooled to -10 °C. The reaction mixture was quickly supplemented by 18.5 mL of BF₃-diethyletherate and stirred until temperature had again reached -10 °C. Subsequently 16.2 mL of isoamyl nitrite was added dropwise under vigorous stirring within 5 min. Stirring and cooling was continued for further 15 min. To the reaction mixture obtained was added 100 mL of diethyl ether under stirring. After 1h, cooling was removed, and an additional 250 mL of diethyl ether were added. A colorless crystalline precipitate of diazonium salt formed. The heterogeneous reaction mixture was kept overnight at 4 °C. The crystals were collected by filtration and washed four times with 100 mL of diethyl ether, each. The crystalline diazonium tetra-fluoroborate obtained was kept under reduced pressure (17 mbar) for 3h. Yield: 27.29 g (78%) of 2,6-dichloro-4-sulfamoylbenzenediazonium tetrafluoroborate (note: the diazonium salt is safe to handle (dec. > 170 °C) and can be stored at 4 °C for several days).

The 2,6-dichloro-4-sulfamoylbenzenediazonium tetrafluoroborate obtained was dissolved under cooling in 130 mL of concentrated aqueous hydrochloric acid. The solution was cooled to -10 °C. Under vigorous stirring a solution of 50.86 g SnCl₂ in 50 mL of concentrated hydrochloric acid was slowly added dropwise to the cooled diazonium tetrafluoroborate solution (duration: 80 min). The temperature was kept below -6 °C during the subsequent addition of the SnCl₂. A colorless precipitate formed. The reaction mixture obtained was kept at 4 °C overnight, and the solid material was collected by filtration and washed with brine until the filtrate was neutral. The free hydrazine base was liberated upon addition of 100 mL of aqueous saturated NaHCO₃, followed by addition of solid NaHCO₃ until gas evolution had ceased. The solid product was extracted with a mixture of 450 mL ethyl acetate and 50 mL of methanol. The organic phase was washed neutral with brine, dried over Na₂SO₄. The solvents were removed under reduced pressure. Overall yield: 11.74 g (44%) of **3,5-dichloro-4-hydrazinylbenzenesulfonamide (6)**

### 4) 4-(5-amino-4-cyano-3-(dimethylamino)-1H-pyrazol-1-yl)-3,5-dichloro-benzenesulfonamide (7) (corresponding to step (e) in the general part of the description)

One-pot synthesis of 4-(5-amino-4-cyano-3-(dimethylamino)-1H-pyrazol-1-yl)-3,5-dichlorobenzene-sulfon-amide (**7**).

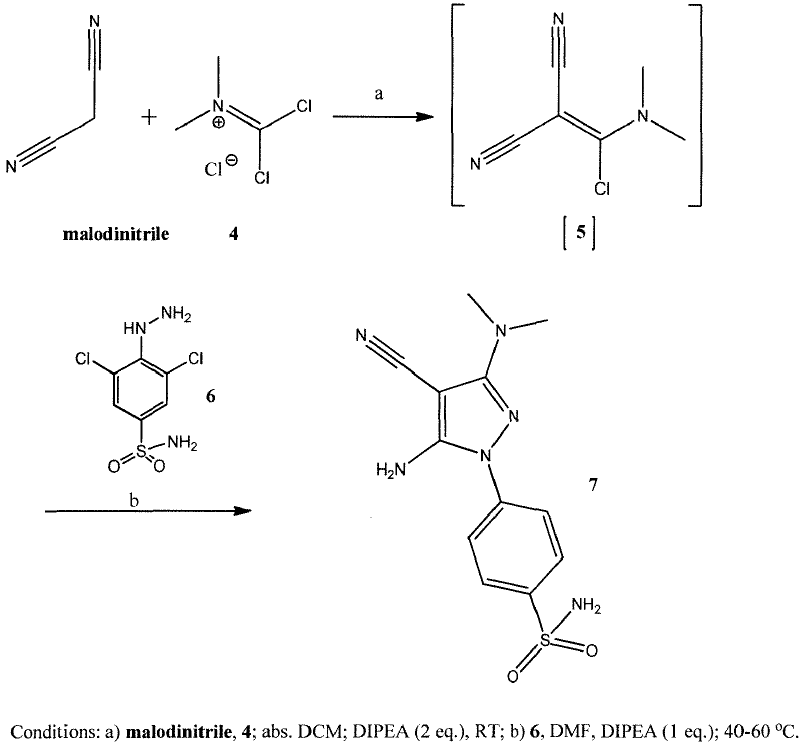

*N*-(Dichloromethylene)-*N*-methylmethanaminium chloride (**4**) (13.32 g; 82 mmol) was transferred into 120 mL of abs. DCM (dichloromethane). To the suspension was added malodinitrile (5.42 g, 82 mmol) dissolved in 50 mL of abs. DCM. The heterogeneous reaction mixture was cooled in an ice bath to 5°C. Under vigorous stirring 21.21 g (2 eq.) of DIPEA (*N,N-*diisopropylethylamine) were added dropwise (within 10 min). The cooling was removed and stirring continued until all iminium chloride **4** had dissolved (duration ca. 40 min). An additional equivalent of DIPEA in DCM was added dropwise. To the orange reaction mixture obtained was added dropwise a solution of 21.00 g (82 mmol) 3,5-dichloro-4-hydrazinylbenzenesulfonamide (**6**) in 120 mL of abs. DMF (dimethyl formamide). Subsequently, DCM was removed under reduced pressure (80 mbar, 50 °C bath temperature). The reaction mixture was kept at 50 °C overnight. The homogeneous solution obtained was diluted with 300 mL of water. The aqueous phase was extracted with 700 mL of ethyl acetate. The organic phase was washed with water, dried over sodium sulfate and evaporated under reduced pressure. During evaporation a colorless precipitate was formed, which was collected by filtration (byproduct). Upon continuation of the ethyl acetate removal additional crystalline material separated. These crystals were collected by filtration and washed with cold ethyl acetate: 8.74 g of pure 7 (TLC). The residual material was subjected to MPLC (silica gel; ethyl acetate/pentane = 3.2/1.8). Fractions containing pure 7 were combined and evaporated at reduced pressure (2.67 g of **7**). Total yield of **4-(5-amino-4-cyano-3-(dimethylamino)-1H-pyrazol-1-yl)-3,5-dichloro-benzene-sulfon-amide (7):** 11.41g (35 %).

### 5) 5-amino-1-(2,6-dichloro-4-sulfamoyl-phenyl)-3 dimethylamino-1H-pyrazole-4-carboxylic acid amide (8) (corresponding to step (d) in the general part of the description)

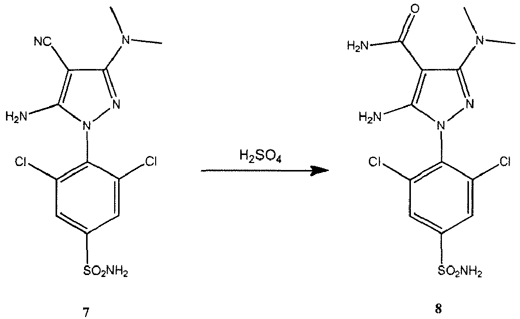

4-(5-Amino-4-cyano-3-dimethylamino-pyrazol-1-yl)-3,5-dichloro-benzenesulfonamide (7) (11.7 g, 0.031 mol) was treated with concentrated sulfuric acid (98%, 40 mL) under stirring at room temperature. To the reaction mixture was slowly added H₂O (2 mL) and stirring was continued overnight. After 16 h additional sulfuric acid (40 mL) was added and stirring was continued for further 16 h. The reaction mixture was added dropwise into a separation funnel containing cooled saturated sodium hydroxide and ethyl acetate. Alkaline pH was continuously restored by addition of further quantities of cold sodium hydroxide solution. Temperature was controlled by addition of ice. The neutralization with sodium hydroxide was stopped at pH 3-5. The final pH of about 8 was achieved with sodium hydrogen carbonate. The organic phase was separated, and the aqueous phase was extracted once with ethyl acetate. The organic phases were combined, dried over magnesium sulfate and concentrated under reduced pressure. The solid material obtained was vigorously stirred in diethyl ether. The yellowish ether phase was removed by filtration. The solid residual collected was washed with diethyl ether and dried in vacuo. Yield: 7.93 g (65 %) of **5-amino-1-(2,6-dichloro-4-sulfamoyl-phenyl)-3 dimethylamino-1H-pyrazole-4-carboxylic acid amide (8).**

### 6) Final assembling of Otviciclib (N-{4-[1-(2,6-dichloro-4-sulfamoyl-phenyl)-3-dimethylamino-4-oxo-4,5-dihydro-1H-pyrazolo[3,4-d]pyrimidin-6-ylmethyl]-phenyl)-2-diethylamino-acetamide) (corresponding to step (a) in the general part of the description)

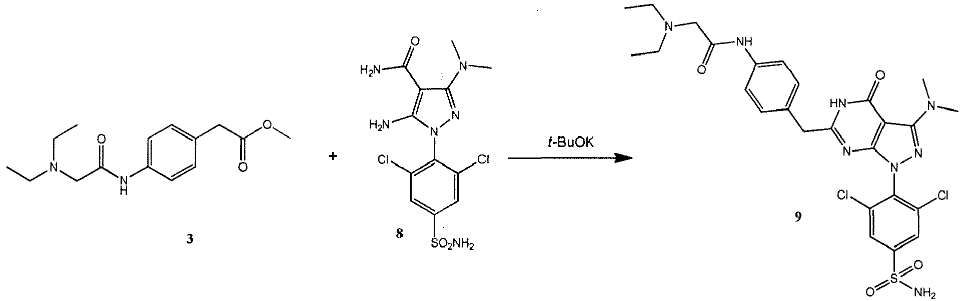

5-amino-1-(2,6-dichloro-4-sulfamoyl-phenyl)-3 dimethylamino-1H-pyrazole-4-carboxylic acid amide (**8**) (13.84 g, 0.035 mol) and [4-(2-diethylamino-acetylamino)-phenyl]-acetic acid methyl ester (**3**) (20.57 g, 2.1 equivalent) were dissolved in dry DMF (50 mL) in an atmosphere of argon. To the thus obtained solution potassium tert-butoxide (19.75 g, 5 equivalents) was added under ice-cooling and stirring. The reaction mixture was heated to 40°C and stirred overnight under argon. The green-brownish reaction mixture was poured into half-saturated sodium chloride solution which contained 25 mL acetic acid. The mixture was extensively extracted with ethyl acetate. Subsequently, the aqueous phase was adjusted to pH 8 by addition of sodium hydrogencarbonate and further extracted with ethyl acetate. The combined organic phases were extensively washed with aqueous sodium chloride, sodium hydrogen carbonate, water and dried over magnesium sulfate. The organic phase was concentrated under reduced pressure. The red oil obtained was subjected to filtration over a silica gel column with ethyl acetate containing 5% of methanol as eluent. This is thus only a filtration step, but not a chromatographic purification step. Product containing fractions were combined and evaporated under reduced pressure. During evaporation crystalline product separated. The primary crystalline material was collected (8.41 g of **9**) and evaporation was continued. A second crop of pure product was obtained (5.63 g). Total yield: 14.04 g (64%) of **N-{4-[1-(2,6-dichloro-4-sulfamoyl-phenyl)-3-dimethylamino-4-oxo-4,5-dihydro-1H-pyrazolo [3,4-d] pyrimidin-6-ylmethyl]-phenyl}-2-diethylamino-acetamide (9)** (*Otviciclib* **9**)*.*

In order to further improve the purity of the thus obtained product, recrystallization from chloroform has been performed.

**HRMS:** calc. for C₂₆H₃₁Cl₂N₈O₄S: 621.1561; found: 621.1564 [M+H]⁺.

**¹H NMR (600.25 MHz, DMSO- d₆, 25°C):** δ = 1.00 (t, ³J = 7.2, 6H, ((C*H*₃CH₂)₂N), 2.58 (q, ³J = 7.2, 4H, ((CH₃C*H*₂)₂N), 3.05 (s, 6H, ((C*H*₃)₂N), 3.12 (s, 2H, *C*H₂-17), 3.81 (s, 2H), 7.23 (m[AA'], 2H, H-12, aniline, correlation to C_{q}-14 [137.30]), 7.56 (m[BB'], 2H, H-13, aniline), 7.80 (s, br, 2H, 4'-SO₂N*H*₂), 8.07 (s, 2H, H-3', sulfonamide), 9.59 (s, 1H, CONH-15), 12.39 (s, vbr, 1H, HN8CO, pyrimidine ring).

**¹³C{¹H}NMR (150.93 MHz, DMSO-d₆, 25°C):** δ = 11.96 (*C*H₃CH₂)₂N), 39.43 (aniline-*C*H₂-C-7 pyrimidine, overlay DMSO, correlation to 7.23), 40.33 ((*C*H₃)₂N-C3 pyrazole ring), 47.88 ((CH₃*C*H₂)₂N), 57.40 (*C*H₂-17), 93.76 (*C*_{q}-4, pyrazole ring), 119.38 (CH-13, aniline), 126.00 (CH-3', sulfonamide), 128.87 (CH-12, aniline), 130.87 (*C*_{q}-11, aniline), 135.43 (*C*_{q}-2', sulfonamide), 135.71 (*C*_{q}-1', sulfonamide), 137.30 (*C*_{q}-14, aniline), 146.75 (*C*_{q}-4', sulfonamide), 155.11 (*C*_{q}-5, pyrazole ring), 155.79 (*C*_{q}-3, pyrazole ring), 157.82 (*C*⁹=O), 160.90 *C*_{q}-7, pyrimidine ring), 169.83 (NH*C¹⁶*=0, glycine part).

### 7) Synthesis of Otviciclib mono-hydrochloride

*Otviciclib* (2.00 g, 3.2 mmol) was dissolved in boiling chloroform (50 mL). To the thus obtained clear solution were added 3 mL of a 4M solution of hydrogen chloride in dioxane under stirring. The solution obtained was cooled in an ice-bath and dry diethyl ether (100 mL) was added. A semi-crystalline precipitate was formed. The solvents were decanted, and 100 mL of fresh dry ether was added so that the colorless precipitate became filterable. The crystals were collected, washed extensively with ether and dried in vacuo. The crystalline material obtained was recrystallized twice from dry methanol. Residual solvent was removed at 100°C (17 mbar). Yield: 1.01 g (48 %) of *Otviciclib* mono-hydrochloride, m.p. 208-210°C.

### NMR of Otviciclib^{∗}HCl

Numbering used for NMR assignment (numbers in square brackets [xy] indicate correlation peaks in HMBC-spectrum):

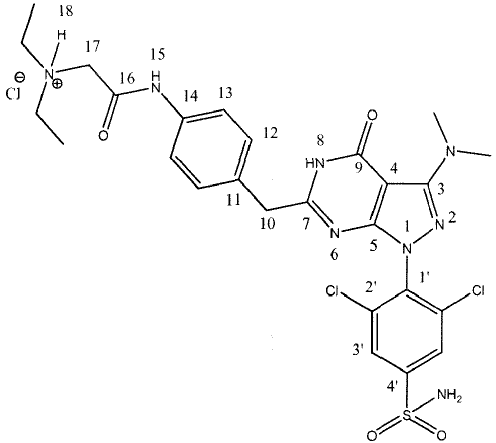

**¹H NMR (600.25 MHz, DMSO-d₆, 25°C): δ** = 1.23 (t, ³*J* = 7.2, 6H, ((C*H*₃CH₂)₂N), 3.04 (s, 6H, ((C*H*₃)₂N), 3.21 (br, 4H, ((CH₃C*H*₂)₂N), (no correlation with any carbon, but COSY with (C*H*₃CH₂)₂N), [3.34 *H*OD], 3.84 (s, br, 2H, aniline-C¹⁰*H*₂-C-7 pyrimidine), [correlation to 39.56, overlap with DMSO], 4.12 (s, br, 2H, C*H*₂-17), 7.27 (m[AA'], 2H, *H*-12, aniline, correlation to C_{q}-14 [136.83]), 7.55 (m[BB'], 2H, *H*-13, aniline, correlation to C_{q}-11 [131.83]), 7.84 (s, 2H, 4'-SO₂N*H*₂), 8.07 (s, 2H, *H*-3', sulfonamide, correlation to C_{q}-1' [135.66]), 9.76 (s, br, 1H, N*H*⁺-18) (coupling to CH₂-17 [4.12] and (CH₃C*H*₂)₂N) [3.21] (no correlation detectable), 10.98 (s, br, 1H, N*H*-15), (no correlation detectable), 12.43 (s, br, 1H, N⁸*H*CO, pyrimidine ring, correlation with [160.81] (C-7, pyrimidine) and [93.77] (C-4, pyrimidine).

**¹³C{¹H}NMR (150.93 MHz, DMSO-d₆, 25°C): δ** = 8.90 (*C*H₃CH₂)₂N), 39.56 (aniline-*C*H₂-C-7 pyrimidine, overlay DMSO, correlation to 3.83), (note: in NMR of free base: *C*H₂-C-7 is recorded as a distinct resonance at 39.52 visible), 40.33 ((*C*H₃)₂N-C3 pyrazole ring), 48.47 ((CH₃*C*H₂)₂N), 53.07 (*C*H₂-17), 93.77 (*C*_{q}-4, pyrazole ring), 119.57 (CH-13, aniline), 126.00 (CH-3', sulfonamide), 129.12 (CH-12, aniline), 131.83 (*C*_{q}-11, aniline), 135.38 (*C*_{q}-2', sulfonamide), 135.66 (*C*_{q}-1', sulfonamide), 136.83 (*C*_{q}-14, aniline), 146.77 (*C*_{q}-4', sulfonamide, weak correlation with H-3' [8.07], 155.08 (*C*_{q}-5, pyrazole ring), 155.78 (*C*_{q}-3, pyrazole ring), 157.78 (*C*⁹=O, pyrimidine ring), 160.81 (*C*_{q}-7, pyrimidine ring), correlation with N⁸H [12.43], 163.31 (NH*C¹⁶*=O, glycine part).

### Elemental analysis:

| **OTVICICLIB x HCI** | **found 1** | **found 2** | **found 3** | **mean found** | **calculated** | **deviation** |
|---|---|---|---|---|---|---|
| **C** | 47.16 | 47.09 | 47.12 | 47.12 | 47.46 | 0.34 |
| **H** | 4.85 | 4.80 | 4.80 | 4.82 | 4.75 | -0.07 |
| **N** | 16.70 | 16.72 | 16.78 | 16.73 | 17.03 | 0.30 |
| **O** | 10.75 | 10.23 | 10.17 | 10.38 | 9.73 | -0.66 |
| **s** | 4.67 | 4.87 | 4.79 | 4.78 | 4.87 | 0.10 |
| **Chloride** | 5.39 | 5.49 | 5.42 | 5.43 | 5.39 | -0.05 |
| **Cl total** | 16.36 | 16.25 | 16.34 | 16.32 | 16.16 | -0.15 |
| **no ash content** | | | | | | |

The compounds of formula (I) obtained by the synthetic approach described herein and as set out in the claims are useful, inter alia, in the prophylaxis and/or treatment of cell proliferative diseases, infectious diseases, pain, asthma, diabetes, neurodegenerative diseases, cardiovascular diseases and inflammation as set out in WO 2018/099952 A1. The present invention, which relates to the synthesis of such compounds, is thus industrially applicable.

## Claims

1. A method of production of a compound represented by the following formula (I) 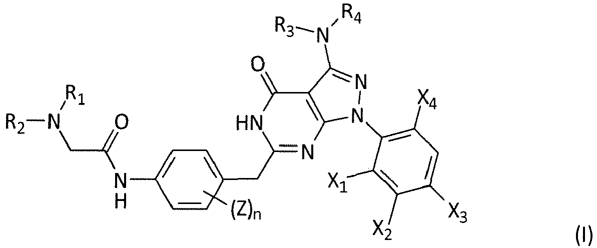 or a salt thereof,
wherein
X₁ is hydrogen, halogen or C₁₋₆-alkoxy,
X₂ is hydrogen or halogen,
X₃ is hydrogen, SO₂NH₂, CONH₂ or COOH,
X₄ is hydrogen, halogen or C₁₋₆-alkoxy,
R₁ and R₂ are independently selected from hydrogen, C₁₋₆-alkyl and C₁₋₆-acyl, or R₁ and R₂ form, together with the nitrogen atom to which both are attached, a 4 to 8-membered aliphatic heterocycle which contains a total of 1 to 3 heteroatoms selected from N, O and S in the ring, and which is optionally substituted with one or more groups independently selected from halogen, C₁₋₆-alkyl, C₁₋₆-haloalkyl and C₁₋₆-acyl,
R₃ and R₄ are independently selected from hydrogen, C₁₋₆-alkyl and C₁₋₆-haloalkyl,
each Z is independently selected from halogen, CN, C₁₋₆-alkyl, C₁₋₆-alkoxy and C₁₋₆-acyl,
n is an integer from 0 to 4,
wherein the method comprises step (a) of reacting a compound of formula (II) 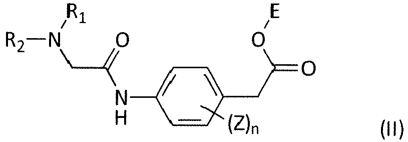
or a salt thereof,
wherein Z, n, R₁ and R₂ as defined for formula (I), and E is C₁₋₆-alkyl,
with a compound of formula (VII) 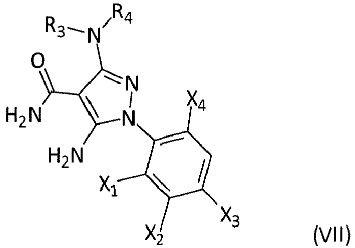
or a salt thereof,
wherein X₁, X₂, X₃, X₄, R₃ and R₄ as defined for formula (I),
to obtain the compound of formula (I) or the salt thereof.

2. The method according to claim 1, wherein in step (a), compounds (II) and (VII) are reacted in the presence of a base in an aprotic solvent, preferably wherein a base is added to a mixture of compounds (II) and (VII) in dimethylformamide.

3. The method according to claim 2, wherein
- the base is an alcoholate, preferably potassium tert-butanolate;
- the reaction of compounds (II) and (VII) is conducted under a protective atmosphere;
- the reaction of compounds (II) and (VII) is conducted at a temperature of 0 to 80°C, preferably 20 to 60°C, for 1 to 48 hours, preferably 1 to 4 hours, and/or
- compound (I) is recrystallized from chloroform.

4. The method according to any one of claims 1 to 3, wherein the method furthermore comprises
(i) the following step (b) of reacting a compound of formula (III) 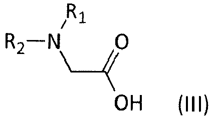
wherein R₁ and R₂ as defined for formula (I),
or a salt thereof,
with a compound of formula (IV) 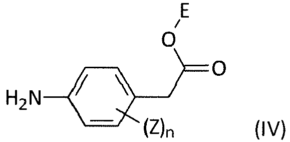
wherein E, Z and n are as defined for formula (II),
or a salt thereof,
to obtain the compound of formula (II) or a salt thereof;
(ii) the following step (c) of reacting a compound of formula (V) 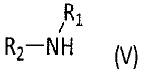
wherein R₁ and R₂ as defined for formula (I),
or a salt thereof,
with a compound of formula (VI) 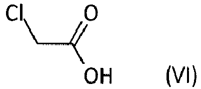
or a salt thereof,
to obtain the compound of formula (III) or the salt thereof; and/or
(iii) the following step (d) of reacting a compound of formula (VIII) 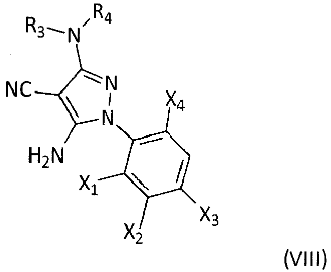
wherein X₁, X₂, X₃, X₄, R₃ and R₄ are as defined for formula (I),
or a salt thereof,
with an acid to obtain the compound of formula (VII) or the salt thereof.

5. The method according to claim 4, wherein in step (d), concentrated sulfuric acid as the acid is added to the compound of formula (VIII), preferably followed by the addition of water, and wherein the reaction is preferably conducted at a temperature of from 0 to 50°C for a duration of 2 to 50 hours, whereafter the reaction mixture is preferably quenched with an aqueous sodium hydroxide solution.

6. The method according to claim 4 or 5, wherein the method furthermore comprises the following step (e) of reacting a compound of formula (IX) 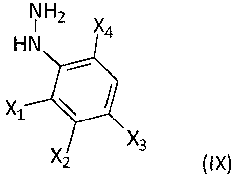
wherein X₁, X₂, X₃ and X₄ are as defined for formula (I),
or a salt thereof,
with a compound of formula (X) 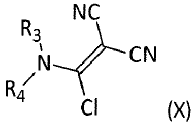
wherein R₃ and R₄ are as defined for formula (I),
or a salt thereof
to obtain the compound of formula (VIII) or the salt thereof.

7. The method according to claim 6, wherein in step (e), the compound of formula (X) is formed as an intermediate by the reaction of propanedinitrile with a compound of formula (XII) 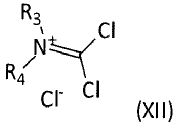 preferably in a solvent containing dichloromethane and diisopropyl ethylamine.

8. The method according to claim 6, wherein the method furthermore comprises the following step (f) of reacting a compound of formula (XIII) 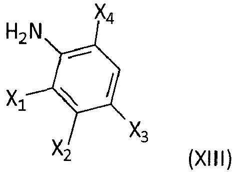
wherein X₁, X₂, X₃ and X₄ are as defined for formula (I),
or a salt thereof,
with a C₁₋₁₀ alkyl nitrite,
followed by reduction,
to obtain the compound of formula (IX) or the salt thereof
wherein in step (f), preferably, the compound of formula (XIII) is reacted with the C₁₋₁₀ alkyl nitrite, which is preferably isoamyl nitrite, and boron trifluoride diethyl etherate, following by the reduction of the thus formed intermediate with Sn(II)Cl₂ in concentrated hydrochloric acid.

9. The method according to any one of the preceding claims, wherein
X₁ is F, Cl, or MeO; preferably F or Cl; more preferably Cl;
X₂ is hydrogen, or F; preferably hydrogen;
X₃ is hydrogen, SO₂NH₂, or CONH₂; more preferably SO₂NH₂, or CONH₂; even more preferably SO₂NH₂; and
X₄ is F, Cl, or MeO; preferably F or Cl; more preferably Cl.

10. The method according to any one of the preceding claims,
wherein R₃ and R₄ are independently selected from C₁₋₆-alkyl and C₁₋₆-haloalkyl; preferably C₁₋₃-alkyl or C₁₋₃-haloalkyl; more preferably C₁₋₃-alkyl; even more preferably methyl or ethyl; still more preferably methyl;
and/or wherein X₁ and X₄ are both chloro, X₂ is hydrogen, X₃ is SO₂NH₂, R₁ and R₂ are both ethyl, R₃ and R₄ are both methyl and n is 0.

11. A compound of formula (II) 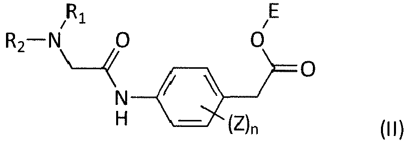
R₁ and R₂ are independently selected from hydrogen, C₁₋₆-alkyl and C₁₋₆-acyl,
or R₁ and R₂ form, together with the nitrogen atom to which both are attached, a 4 to 8-membered aliphatic heterocycle which contains a total of 1 to 3 heteroatoms selected from N, O and S in the ring, and which is optionally substituted with one or more groups independently selected from halogen, C₁₋₆-alkyl, C₁₋₆-haloalkyl and C₁₋₆-acyl,
each Z is independently selected from halogen, CN, C₁₋₆-alkyl, C₁₋₆-alkoxy and C₁₋₆-acyl; n is an integer from 0 to 4, and
E is C₁₋₆-alkyl.

12. The method according to any one of claims 1 to 10 or the compound according to claim 11,
wherein each Z is independently selected from halogen, CN, C₁₋₃-alkyl, C₁₋₃-alkoxy and C₁₋₃-acyl; preferably halogen, CN, C₁₋₃-alkyl and C₁₋₃-alkoxy; more preferably halogen, CN and C₁₋₃-alkyl; even more preferably halogen, CN and methyl, still more preferably halogen; and/or
wherein n is an integer from 0 to 3; preferably from 0 to 2; more preferably 0 or 1; even more preferably 0.

13. The compound according to claim 11 or 12, wherein E is C₁₋₃-alkyl, preferably methyl or ethyl, more preferably methyl; and/or wherein R₁ and R₂ are both ethyl, n is 0, and E is methyl.

14. The method according to any one of claims 1 to 10, 12 and 13 or the compound according to any one of claim 11 to 13,
(i) wherein R₁ and R₂ are independently selected from C₁₋₆-alkyl; preferably C₁₋₃-alkyl; more preferably methyl, ethyl or 2-propyl; even more preferably ethyl; or
(ii) wherein R₁ and R₂ form, together with the nitrogen atom to which both are attached, a 5 or 6-membered aliphatic heterocycle which contains a total of 1 to 3 heteroatoms selected from N and O in the ring, and which is optionally substituted with one or more groups independently selected from halogen and C₁₋₆-alkyl;
R₁ and R₂ preferably form, together with the nitrogen atom to which both are attached, a 6-membered aliphatic heterocycle which contains a total of 1 or 2 heteroatoms selected from N and O in the ring, and which is optionally substituted with one or more independently selected from C₁₋₆-alkyl;
R₁ and R₂ more preferably form, together with the nitrogen atom to which both are attached, a piperidine, piperazine or morpholine ring, and each of which is optionally substituted with one or more independently selected from C₁₋₆-alkyl.

15. A method of production of a compound of formula (VIII) 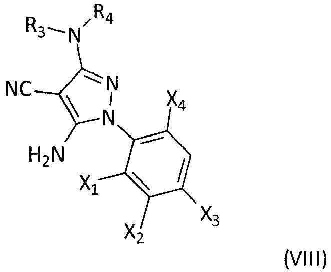 wherein
X₁ is hydrogen, halogen or C₁₋₆-alkoxy,
X₂ is hydrogen or halogen,
X₃ is hydrogen, SO₂NH₂, CONH₂ or COOH,
X₄ is hydrogen, halogen or C₁₋₆-alkoxy,
R₃ and R₄ are independently selected from hydrogen, C₁₋₆-alkyl and C₁₋₆-haloalkyl, or a salt thereof,
comprising the step (t) of reacting propanedinitrile with a compound of formula (XII) 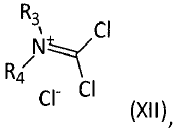 preferably in a solvent containing dichloromethane and diisopropyl ethylamine, to form a compound of formula (X) 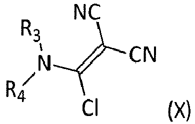
wherein R₃ and R₄ are as defined for formula (VIII),
or a salt thereof;
and step (u) of reacting the compound of formula (X) or the salt thereof with a compound of formula (IX) 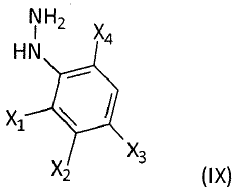
wherein X₁, X₂, X₃ and X₄ are as defined for formula (VIII),
or a salt thereof,
to obtain the compound of formula (VIII) or the salt thereof.
